# EUROPÄISCHE PATENTANMELDUNG

(11) **EP 2 335 565 A1**
(43) Veröffentlichungstag der Anmeldung: **22.06.2011**
(21) Anmeldenummer: 09179802.5
(22) Anmeldetag: 18.12.2009
(51) Int. Cl.: A61B 5/00

(54) **Schutzbehälter für Aufnahme wieder verwendbarer diagnostischer Komponenten**

(71) Anmelder: Roche Diagnostics GmbH, 68305 Mannheim (DE); F. Hoffmann-La Roche AG, 4070 Basel (CH)
(72) Erfinder: Heck, Wolfgang, 67227 Frankenthal (DE); Roesicke, Bernd, 68305 Mannheim (DE); Frisch, Gerhard, 68535 Edingen-Neckarhausen (DE)
(74) Vertreter: Stößel, Matthias

(57) **Zusammenfassung**

Es wird ein Schutzbehälter (158) zur Aufnahme eines wieder verwendbaren Steuerteils (118) eines transkutanen Sensorsystems (110) zum Nachweis mindestens eines Analyten in einer Körperflüssigkeit vorgeschlagen. Das Steuerteil (118) umfasst mindestens einen Anschluss (142), welcher mindestens einen Sensoranschluss (148) zur Verbindung mit mindestens einem transkutanen Sensor (112) aufweist. Der Schutzbehälter (158) weist mindestens ein Behältergehäuse (160) auf. Das Steuerteil (118) ist in dem Behältergehäuse (160) aufnehmbar. Das Behältergehäuse (160) ist eingerichtet, um das Steuerteil (118) gegenüber Umwelteinflüssen abzuschirmen. Das Behältergehäuse (160) weist weiterhin mindestens einen Verbinder (164) auf, welcher mit dem Anschluss (142) verbindbar ist und diesen mediendicht verschließt.

## Beschreibung

### Gebiet der Erfindung

Die Erfindung betrifft einen Schutzbehälter zur Aufnahme eines wieder verwendbaren Steuerteils eines transkutanen Sensorsystems. Weiterhin betrifft die Erfindung ein Kit, umfassend einen Schutzbehälter sowie ein wieder verwendbares Steuerteil, und eine Sensorvorrichtung zum Nachweis mindestens eines Analyten in einer Körperflüssigkeit. Schließlich betrifft die Erfindung ein Verfahren zum Schutz eines wieder verwendbaren Steuerteils eines transkutanen Sensorsystems zum Nachweis mindestens eines Analyten. Derartige Vorrichtungen und Verfahren werden insbesondere im Bereich der medizinischen Diagnostik eingesetzt, beispielsweise in Krankenhäusern, Pflegeeinrichtungen oder im privaten Umfeld, um einen oder mehrere Analyte wie beispielsweise Glucose, Lactat, Cholesterin, Triglyceride oder andere Arten von Analyten qualitativ oder quantitativ in einer oder mehreren Körperflüssigkeiten, wie beispielsweise interstitieller Flüssigkeit oder Blut, nachzuweisen.

### Stand der Technik

Die Überwachung bestimmter Körperfunktionen, insbesondere die Überwachung einer oder mehrerer Konzentrationen bestimmter Analyte, spielt bei der Vorbeugung und Behandlung verschiedener Krankheiten eine wesentliche Rolle. Ohne Beschränkung weiterer möglicher Anwendungen wird die Erfindung im Folgenden unter Bezugnahme auf eine Blutglucoseüberwachung beschrieben. Grundsätzlich ist die Erfindung jedoch auf andere Arten von Analyten und/oder die Überwachung anderer Arten von Körperfunktionen übertragbar, so dass auch beispielsweise, alternativ oder zusätzlich zu einem qualitativen und/oder quantitativen Nachweis eines Analyten in der Körperflüssigkeit die Überwachung einer anderen Art von Körperfunktion sinngemäß unter den Begriff "Nachweis mindestens eines Analyten in einer Körperflüssigkeit" subsummiert werden kann.

Neben so genannten Punktmessungen, bei welchen einem Benutzer gezielt eine Probe einer Körperflüssigkeit entnommen und auf die Analytkonzentration untersucht wird, etablieren sich zunehmend auch kontinuierliche Messungen. So etabliert sich beispielsweise eine kontinuierlich Glucosemessung im Interstitium (auch als Continuous Monitoring, CM, bezeichnet) in jüngerer Vergangenheit als mit wichtige Methode zum Management, zur Überwachung und zur Steuerung beispielsweise eines Diabetes-Status. Vorerst beschränkt sich diese kontinuierliche Überwachung in vielen Fällen auf Diabetiker vom Typ I, also Diabetiker, welche üblicherweise auch eine Insulinpumpe tragen. Mittlerweile kommen dabei in der Regel direkt implantierte elektrochemische Sensoren zum Einsatz, welche häufig auch als nadelartige Sensoren (Needle Type Sensors, NTS) bezeichnet werden. Dabei wird der aktive Sensorbereich direkt an den Messort gebracht, welcher in der Regel im interstitiellen Gewebe angeordnet ist, und beispielsweise unter Verwendung eines Enzyms (beispielsweise Glucoseoxidase, GOD) Glucose in elektrische Ladung umsetzt, die im Verhältnis zur Glucose-Konzentration steht und als Messgröße verwendet werden kann. Beispiele derartiger transkutaner Messsysteme sind in US 6,360,888 B1 oder in US 2008/0242962 A1 beschrieben.

Akutelle Continuous Monitoring-Systeme sind somit in der Regel transkutane Systeme. Dies bedeutet, dass der eigentliche Sensor unterhalb der Haut des Benutzers angeordnet ist. Ein Auswerte- und Steuerteil des Systems (auch als Patch bezeichnet) befindet sich jedoch in der Regel außerhalb des Körpers des Benutzers, also außerhalb des menschlichen oder tierischen Körpers. Der Sensor wird dabei in der Regel mittels eines Insertionsbestecks appliziert, welches exemplarisch ebenfalls in US 6,360,888 B1 beschrieben wird. Auch andere Arten von Insertionsbestecken sind bekannt. Die Tragedauer eines Sensors beträgt in der Regel ca. eine Woche. Danach lassen in der Regel Einflüsse, wie beispielsweise ein Enzymverbrauch und/oder eine Abkapselung im Körper, die Sensitivität des Sensors abfallen, oder es ist ein Ausfall des Sensors zu erwarten. Die Verlängerung der Tragedauer stellt ein aktuelles Entwicklungsgebiet dar. Dies bedeutet jedoch, dass der Sensor und optional mit ihm unmittelbar in Verbindung stehende Komponenten, wie beispielsweise eine Insertionsnadel, als austauschbare Bauelemente ausgestaltet werden sollten. Dementsprechend stellen der Sensor und optional weitere austauschbare Komponenten in der Regel ein so genanntes Einwegteil (Disposable) dar. Das Auswerte- und Steuerteil des Systems wird hingegen in den meisten Fällen wieder verwendet. Dementsprechend ist dieses Auswerte- und Steuerteil in der Regel als so genanntes wieder verwendbares Teil (Reusable) ausgestaltet.

Durch diese Unterteilung des transkutanen Sensorsystems in mindestens ein Disposable und mindestens ein Reusable entsteht jedoch prinzipiell das Problem, dass zwischen dem Auswerte- und Steuerteil (Patch) und dem Sensor eine lösbare Schnittstelle benötigt wird, die, je nach Ausführungsform, in der Regel mehr oder weniger dem Zugriff des Benutzers ausgesetzt ist. Diese Schnittstelle ist in der Regel auch insofern erforderlich, da aus Biokompatibilitätsgründen und Hygienegründen nach der Herstellung des implantierbaren Sensors eine Sterilisation dieses Sensors erforderlich ist und da in der Regel nach Gebrauch des Reusables eine Desinfektion desselben erforderlich ist.

Für die Sterilisation des Sensors kommen grundsätzlich verschiedene Konzepte in Betracht. Da bei den derzeit verwendeten Konzepten jedoch der Sensor, beispielsweise eine Sensorchemie, mit der Körperflüssigkeit, beispielsweise dem Interstitium, fluidisch Kontakt haben soll, sind sowohl eine chemische als auch eine thermische Sterilisation in der Regel ausgeschlossen. Eine chemische Sterilisation, wie beispielsweise mittels Ethylenoxid, könnte in Spuren in der Sensorchemie zurückbleiben, was später im implantierten Zustand zu einem Eintrag des Sterilisationsmittels in das Körpergewebe führen könnte. Eine thermische Sterilisation hingegen könnte eine empfindliche Sensorchemie, wie beispielsweise Enzyme, zerstören. Somit bleibt in der Regel lediglich eine Strahlensterilisation, beispielsweise mittels Betastrahlung, als Verfahren zur Sterilisation des Sensors übrig. Strahlendosen in der benötigten Stärke schädigen jedoch in vielen Fällen Elektronikkomponenten. Eine partielle Schirmung dieser Komponenten, wie sie beispielsweise in EP 1 178 841 B beschrieben wird, erfordert jedoch einen hohen Aufwand im Herstellungsprozess.

Viele derzeit verfolgte Ansätze für die kontinuierliche Überwachung von Analytkonzentrationen sehen vor, dass ein so genanntes Bodymount mittels eines Pflasters auf eine Haut des Benutzers aufgeklebt wird. Ein Beispiel eines derartigen Systems ist in US 2008/024962 A1 und EP 1 972 275 A1 beschrieben. Das Bodymount ist ebenfalls als Disposable ausgelegt. Es enthält eine Batterie, ein elektronisches Speichermedium (beispielsweise ein EEPROM und/oder ein Flash-EPROM), ein oder mehrere Halteelemente für den eigentlichen Sensor, mindestens eine Steckverbindung sowie mindestens ein Loch in einer Bodenplatte, durch welches der Sensor, beispielsweise mittels einer Insertionsvorrichtung, in das Gewebe des Benutzers hineingeführt wird. Im Speichermedium werden chargenspezifische Sensordaten gespeichert, denn der Sensor und das Bodymount bilden als Disposable in der Regel eine Verpackungseinheit. Weiterhin können Betriebsmodi dort hinterlegt werden. Mittels einer Insertionshilfe wird der Sensor durch das Loch im Bodymount in die Haut insertiert. Die Insertionshilfe befestigt bei der Insertion gleichzeitig den mit dem Stecker versehenen Sensor am Bodymount, so dass nun das am Körper befindliche Bodymount mit zwei festen Steckersystemen versehen ist, nämlich einem Stecker zur Anbindung des Sensors und einem Stecker zur Anbindung des Reusables. Nach der Insertion wird die Insertionshilfe wieder entfernt. Dabei und anschließend austretende Flüssigkeit, beispielsweise Blut oder Interstitium, kann insbesondere mittels eines Tupfers entfernt werden. Anschließend wird das so genannte Reusable aufgesteckt und fixiert. Das Reusable besitzt zwei zu den Anschlüssen des Bodymounts korrespondierende Buchsen. Über diese wird eine elektrische Verbindung zur Batterie, dem Speichermedium und den Sensorelektroden hergestellt und bei Kontakt die Messfunktion des Continuous Monitoring-Systems gestartet. Dieses System muss, bis auf die Sensorelektroden, während des Betriebs in der Regel galvanisch hermetisch isoliert werden, um Leckströme und damit Messfehler zu vermeiden.

Bei derartigen Systemen treten jedoch einige technische Herausforderungen auf. So dürfen potentiostatische elektrochemische Systeme in der Regel nur sehr geringe Leckströme aufweisen, beispielsweise an einer Referenzelektrode des Sensors, da Ströme im Picoampere-Bereich bereits die Referenzelektrode chemisch zersetzen können. Dementsprechend kann das elektrochemische System gegebenenfalls vorzeitig ausfallen. Leckströme von oder zur Arbeitselektrode des elektrochemischen Sensors führen hingegen zu nicht erkennbaren Messfehlern. Daher sind in der Regel die elektrischen Leitungen und Kontakte im System, mit Ausnahme der einzelnen Sensor-Elektroden, galvanisch vollständig zu isolieren. Im geforderten Strombereich ist dies in der Regel nur mit erheblichem Isolationsaufwand bedingt möglich. In das System eindringende, umweltbedingte Störfaktoren, wie beispielsweise Feuchte, Staub, Salze oder ähnliche Umwelteinflüsse, können parasitäre Leckströme verursachen. Das System ist daher nach Möglichkeit gegen derartige Umwelteinflüsse zu schützen. Bei vorliegenden Konzepten sind die Steckerbereiche jedoch notwendigerweise zugänglich, und es herrschen in vielen Fällen Lagerbedingungen, die der Anforderung nach Hochohmigkeit (beispielsweise Widerständen von mehr als 10⁹ Ohm) zuwiderlaufen. Die Hochohmigkeit kann über einen längeren Verwendungszeitraum ohne gezielte Schutzmaßnahmen somit in vielen Fällen nicht prinzipiell gewährleistet werden. Einige graduelle oder unbemerkte schleichende Veränderung der Widerstandsverhältnisse im laufenden, bestimmungsgemäßen Betrieb, kann bei den vorliegenden Signalgrößen daher nur mit großem messtechnischem Aufwand festgestellt werden. Würde eine Fehlerdetektion bei mit dem Reusable zusammenarbeitenden Sensor jedoch in ein Fehlerkonzept (Failsafe Concept) einbezogen werden, würde dies bei zu sensibler Diskriminierung zu unberechtigten Fehlermeldungen führen bzw. wäre bei unzureichender Diskriminierung wirkungslos.

Die Problematik konzentriert sich somit in der Regel auf die Seite des Reusables. Wie oben dargestellt, sehen bekannte Konzepte vor, dass eine Mehrfachanwendung oder sogar eine Anwendung durch mehrere Benutzer erfolgt. Dies bedeutet jedoch, dass das Reusable oft und nicht durch das System kontrollierbar von einem Benutzer gehandhabt wird. Auf Seiten des Sensors selbst ist das Problem hingegen weniger stark ausgeprägt, da dieser Sensor in der Regel Teil des Disposables ist. Dies bedeutet, dass der Sensor durch konstruktive Vorkehrungen so gestaltet sein kann, dass er dem Zugriff des Benutzers nur minimal ausgesetzt ist. Beeinträchtigungen bei Produktion, Lagerung und Transport können durch kontrollierte Prozesse vermieden werden. Beispielsweise ist in US 6,360,888 B1 ein Sensorverpackungssystem zum Lagern und Transportieren eines Glucosesensors bekannt. In diesem Verpackungssystem kann auch ein Indikator vorgesehen sein, welcher überwacht, in welchem Maße der Sensor einer Temperaturbelastung ausgesetzt ist. Auch aus WO 2006/133305 A2 ist eine Schutzverpackung für einen implantierbaren Biosensor bekannt. Grundsätzlich sind auch aus anderen Bereichen Schutzkonzepte bekannt, wie beispielsweise aus US 4,801,271, wonach elektrische Verbindungen vorübergehend mit einem Dummy-Stecker verschlossen werden können.

Derzeitige Konzepte sehen beispielsweise vor, dass der Sensor nach der Produktion in eine Insertions-Hohlnadel eingelegt und in einer Umverpackung sterilisiert wird. Damit ist der Sensor, einschließlich seines Kontaktbereichs, bei bestimmungsgemäßem Gebrauch vor einer Berührung geschützt. Außerdem werden Feuchte, Staub und ähnliche Umwelteinflüsse durch geeignete Verpackungsmaßnahmen während der Lagerung und des Transports ausgeschlossen.

Die beschriebene Problematik liegt also überwiegend auf der Seite des Reusables. Es muss sichergestellt werden, dass das Reusable nach der Herstellung, während des Transports, bei einer Zwischenlagerung und bei Austausch der Disposable-Komponenten, ausreichend gegenüber Umwelteinflüssen geschützt ist. Zwar wäre es grundsätzlich auch möglich, diese Problematik durch eine Umdefinition eines Reusables zu einem Disposable zu umgehen. Da das Reusable jedoch in der Regel eine umfangreiche Elektronik enthält, wäre dies bei höheren Stückzahlen keine wirtschaftliche Lösung der beschriebenen Problematik.

### Aufgabe der vorliegenden Erfindung

Es ist daher eine Aufgabe der vorliegenden Erfindung, Maßnahmen bereitzustellen, welche die Nachteile bekannter transkutaner Sensorsysteme vermeiden. Insbesondere soll mittels der vorgeschlagenen Maßnahmen ein Schutz des Reusables während eines Transports, einer Zwischenlagerung oder während eines Austauschs der Disposable-Komponenten gewährleistet sein.

### Offenbarung der Erfindung

Diese Aufgabe wird gelöst durch einen Schutzbehälter, ein Kit, eine Sensorvorrichtung und ein Verfahren mit den Merkmalen der unabhängigen Patentansprüche. Vorteilhafte Weiterbildungen der Erfindung, welche einzeln oder in beliebiger Kombination realisierbar sind, sind in den abhängigen Ansprüchen dargestellt.

In einem ersten Aspekt der vorliegenden Erfindung wird ein Schutzbehälter zur Aufnahme eines wieder verwendbaren Steuerteils eines transkutanen Sensorsystems zum Nachweis mindestens eines Analyten in einer Körperflüssigkeit vorgeschlagen. Unter einem Schutzbehälter ist dabei eine Vorrichtung zu verstehen, welche mindestens ein Behältergehäuse mit mindestens einem Innenraum aufweist, in welche das Steuerteil ganz oder teilweise einbringbar ist. Dabei ist vorzugsweise in einen Schutzbehälter genau ein Steuerteil einbringbar. Auch eine Anordnung, bei welcher mehrere Steuerteile in einen Schutzbehälter eingebracht werden können, ist jedoch denkbar. Das Behältergehäuse umschließt vorzugsweise das Steuerteil vollständig. Auch eine Ausgestaltung, bei welcher das Behältergehäuse das Steuerteil nicht vollständig umschließt, bei welcher beispielsweise ein Gehäuse des Steuerteils selbst Teil der Abschirmung empfindlicher Teile des Steuerteils ist, ist denkbar. Der Schutzbehälter kann beispielsweise verschließbar ausgestaltet sein und kann beispielsweise eine Behälterklappe, eine Öffnung, ein Schieber oder eine andere Verschlussvorrichtung aufweisen, so dass nach dem Einbringen des Steuerteils ein Verschluss des Innenraums stattfinden kann.

Unter einem transkutanen Sensorsystem ist, wie oben ausgeführt, ein Sensorsystem zu verstehen, bei welchem mindestens ein Sensor vollständig oder teilweise innerhalb eines Körpergewebes eines menschlichen oder tierischen Benutzers angeordnet ist, und mindestens eine Komponente des Sensorsystems vollständig oder teilweise außerhalb des Körpergewebes, wobei die mindestens eine Komponente außerhalb des Körpergewebes, beispielsweise das wieder verwendbare Steuerteil, und der Sensor, durch die Haut oder eine Gewebeoberfläche des Benutzers hindurch miteinander in Verbindung stehen, vorzugsweise über eine drahtgebundene Verbindung, wobei jedoch, alternativ oder zusätzlich, auch eine drahtlose Verbindung möglich ist.

Unter einem Steuerteil ist dementsprechend, wie unten noch näher ausgeführt wird, eine Komponente des transkutanen Sensorsystems zu verstehen, welche eingerichtet ist, um den Sensor anzusteuern und/oder Signale des Sensors aufzunehmen und vollständig oder teilweise auszuwerten.

Wie oben ausgeführt, kann es sich bei dem mindestens einen nachzuweisenden Analyten, wobei auch mehrere Analyte gleichzeitig nachgewiesen werden können, beispielsweise um Blutglucose, Cholesterin, Lactat, Triglyceride oder Kombinationen der genannten und/oder anderer Analyte handeln. Auch ein Nachweis anderer Arten von Analyte ist möglich, wobei der Begriff des Analyten beispielsweise mindestens einen Metaboliten umfassen kann, welcher qualitativ und/oder quantitativ nachgewiesen werden kann. Grundsätzlich ist jedoch auch der Nachweis beliebiger anderer Arten von Körperfunktionen möglich, so dass der Begriff des Analytnachweises allgemein im Rahmen der vorliegenden Erfindung auch beliebige diagnostische Funktionen zur Erfassung mindestens eines Körperzustandes des Benutzers umfassen kann. Bei der Körperflüssigkeit kann es sich beispielsweise um Blut, interstitielle Flüssigkeit oder andere Arten von Körperflüssigkeit handeln.

Das Steuerteil, welches in den Schutzbehälter einbringbar ist, soll mindestens einen Anschluss umfassen. Unter einem Anschluss ist grundsätzlich eine beliebige Schnittstelle zu verstehen, über welche das Steuerteil, vorzugsweise drahtgebunden, mit mindestens einer weiteren Komponente verbindbar ist. Beispielsweise kann der Anschluss, wie unten noch näher ausgeführt wird, mindestens einen Steckanschluss umfassen, also einen Anschluss, welcher über eine Steckverbindung mit mindestens einer weiteren Komponente elektrisch und vorzugsweise auch mechanisch koppelbar ist.

Der Anschluss umfasst mindestens ein Sensoranschluss zur Verbindung des Steuerteils mit mindestens einem transkutanen Sensor. Beispielsweise kann dieser Sensoranschluss einen Sensorstecker umfassen, welcher beispielsweise als männlicher oder weiblicher Stecker ausgestaltet sein kann und/oder welcher beispielsweise eine männliche und/oder eine weibliche Steckerkomponente umfassen kann. Auch andere Ausgestaltungen sind jedoch grundsätzlich möglich.

Der Schutzbehälter soll mindestens ein Behältergehäuse aufweisen. Wie oben beschrieben, kann das Behältergehäuse beispielsweise mindestens einen vollständig oder teilweise geschlossenen Innenraum aufweisen. Das Behältergehäuse kann beispielsweise starr ausgestaltet sein, also dergestalt, dass dieses seine Form zumindest unter Einfluss seiner eigenen Gewichtskraft nicht oder nur unwesentlich verändert. Beispielsweise kann das Behältergehäuse aus einem Kunststoff und/oder einem metallischen Material gefertigt sein. Das Behältergehäuse kann beispielsweise als Container, als Box oder auf ähnliche Weise ausgestaltet sein und kann eine oder mehrere Behältergehäuse-Komponenten umfassen. Das Steuerteil soll in dem Behältergehäuse aufnehmbar sein. Beispielsweise kann das Behältergehäuse, wie oben ausgeführt, zu diesem Zweck einen oder mehrere vollständig oder teilweise geschlossene Innenräume umfassen, in welche das Steuerteil vollständig oder teilweise einbringbar ist. Das Behältergehäuse ist eingerichtet, um das Steuerteil gegenüber Umwelteinflüssen abzuschirmen. Unter einer Abschirmung ist dabei eine Verzögerung des Zutritts der Umwelteinflüsse zum Steuerteil zu verstehen, vorzugsweise eine vollständige Abschottung vor diesen Umwelteinflüssen. Insbesondere kann der Zutritt der Umwelteinflüsse zu dem Steuerteil durch das Behältergehäuse um mindestens einen Faktor 10, vorzugsweise um mindestens einen Faktor 100 und besonders bevorzugt um mindestens einen Faktor 1000 verlangsamt werden. Bei Umwelteinflüssen kann es sich insbesondere um Umwelteinflüsse in Form von Staub, Schmutz, Feuchtigkeit oder Kombinationen der genannten und/oder anderer Arten von Umwelteinflüssen handeln, welche insbesondere den mindestens einen Anschluss angreifen können. Die Abschirmung kann dementsprechend vollständig erfolgen, so dass das gesamte Steuerteil gegenüber den Umwelteinflüssen abgeschirmt ist, wobei jedoch auch eine lediglich teilweise Abschirmung möglich ist. Bei einer lediglich teilweisen Abschirmung kann beispielsweise das Behältergehäuse derart ausgestaltet sein, dass dieses zumindest den mindestens einen Anschluss gegenüber den Umwelteinflüssen abschirmt. Besonders bevorzugt ist es jedoch, wie oben ausgeführt, wenn das Behältergehäuse das gesamte Steuerteil vollständig umschließt und vorzugsweise hermetisch gegenüber den Umwelteinflüssen abschirmt.

Das Behältergehäuse weist weiterhin mindestens einen Verbinder auf. Unter einem Verbinder ist dabei ein Element zu verstehen, welches zumindest mechanisch mit dem mindestens einen Anschluss verbindbar ist und vorzugsweise auch elektrisch. Der Verbinder soll mit dem Anschluss verbindbar sein und diesen mediendicht verschließen. Unter einem mediendichten Verschließen ist dabei ein Verschluss des Verbinder zu verstehen, bei welchem ein Zutritt von Medien wie Staub und Feuchtigkeit im Vergleich zu einem unverschlossenen Anschluss zumindest erheblich verlangsamt ist, vorzugsweise um mindestens einen Faktor 10, insbesondere mindestens einen Faktor 100 und besonders bevorzugt um mindestens einen Faktor 1000. Dementsprechend kann der Verbinder beispielsweise, wie unten noch näher ausgeführt wird, mindestens einen Steckverbinder umfassen, welcher mit dem Anschluss verbunden werden kann und diesen abdichtet, so dass die Medien nicht in den Anschluss eindringen können.

Wie oben ausgeführt, umfasst der Anschluss des Steuerteils zumindest den mindestens einen Sensoranschluss zur Verbindung des Steuerteils mit dem mindestens einen transkutanen Sensor. Darüber hinaus kann der Anschluss des Steuerteils auch mindestens einen weiteren Anschluss umfassen, beispielsweise einen Steueranschluss zur Verbindung des Steuerteils mit mindestens einer Steuerkomponente, beispielsweise mindestens einer Einweg-Steuerkomponente. So kann, wie unten noch näher ausgeführt wird, ein Messsystem zum Nachweis mindestens eines Analyten in einer Körperflüssigkeit beispielsweise das mindestens eine wieder verwendbare Steuerteil, den mindestens einen transkutanen Sensor und mindestens eine Steuerkomponente, beispielsweise mindestens eine Einweg-Steuerkomponente, aufweisen. Die Steuerkomponente kann im zusammengesetzten Zustand beispielsweise mechanisch mit dem transkutanen Sensor gekoppelt sein. Weiterhin kann die Steuerkomponente im zusammengesetzten Zustand des Messsystems beispielsweise mindestens einen Datenspeicher umfassen, beispielsweise einen Datenspeicher zur Speicherung charakteristischer Daten des transkutanen Sensors wie beispielsweise Chargeninformationen und/oder Kalibrationsdaten. Auf diese Weise können die mindestens eine Steuerkomponente und der transkutane Sensor beispielsweise gemeinsam als Einweg-Komponente gelagert und geliefert werden, damit diese dann mit einem wieder verwendbaren Steuerteil zu einem Messsystem verbunden werden können. Die Steuerkomponente kann, alternativ oder zusätzlich zu dem mindestens einen Datenspeicher, beispielsweise einem flüchtigen und/oder einem nicht-flüchtigen Datenspeicher, weiterhin mindestens eine elektrische Energieversorgung umfassen, insbesondere einen elektrischen Energiespeicher, beispielsweise mindestens eine Batterie und/oder mindestens einen Akkumulator. Dementsprechend kann beispielsweise das Steuerteil aus der Steuerkomponente mit elektrischer Energie versorgt werden, wobei die Energieversorgung beispielsweise über den Steueranschluss erfolgt.

Enthält der Anschluss mindestens einen Steueranschluss und mindestens einen Sensoranschluss, so kann der Verbinder, wie oben ausgeführt, mindestens einen Sensorverbinder zum Verbinden mit dem Sensoranschluss und vorzugsweise zum mediendichten Verschließen des Sensoranschlusses sowie weiterhin mindestens einen Steuerverbinder zum Verbinden mit dem Steueranschluss und vorzugsweise zum mediendichten Verschließen des Steueranschlusses aufweisen. Der Steueranschluss und der Sensoranschluss bzw. der Steuerverbinder und der Sensorverbinder sind vorzugsweise als getrennte Komponenten ausgestaltet, beispielsweise als getrennte Steckkomponenten, insbesondere als getrennte Steckverbinder. Grundsätzlich können Steueranschluss und Sensoranschluss und/oder Steuerverbinder und Sensorverbinder jedoch auch ganz oder teilweise zusammengefasst sein.

Die Verbindung des Verbinders mit dem Anschluss kann insbesondere während des Einbringens des Steuerteils in den Schutzbehälter erfolgen. Dies bedeutet, dass beim Einbringen des Steuerteils in den Schutzbehälter gleichzeitig die Verbindung zwischen dem Verbinder und dem Anschluss hergestellt werden kann, beispielsweise die Steckverbindung. Dies bedeutet insbesondere, dass das Steuerteil in den Schutzbehälter, beispielsweise einen Innenraum des Behältergehäuses, einsteckbar sein kann, wobei beim Einstecken gleichzeitig die Verbindung zwischen dem Verbinder und dem Anschluss hergestellt werden kann. Auch eine andere Ausgestaltung ist jedoch grundsätzlich möglich.

Der Schutzbehälter kann insbesondere, wie oben ausgeführt, eine feuchtigkeitsdichte Abschirmung für das in den Schutzbehälter vollständig oder teilweise eingebrachte Steuerteil, also für das ganze Steuerteil oder lediglich einen Teil des Steuerteils, bereitstellen. Insbesondere kann eine feuchtigkeitsdichte Abschirmung für den mindestens einen Anschluss bereitgestellt werden. Um diese Abschirmung bereitstellen zu können, kann einerseits der oben beschriebene Verbinder bereitgestellt werden. Andererseits kann der Schutzbehälter, insbesondere das Behältergehäuse, zusätzlich mindestens einen Verschluss umfassen, beispielsweise mindestens einen Deckel und/oder mindestens einen Schieber, welcher nach Einbringen des Steuerteils in den Innenraum des Behältergehäuses verschließbar ist. Unter einer feuchtigkeitsdichten Abschirmung ist dabei insbesondere zu verstehen, dass ein Eindringen von Feuchtigkeit, insbesondere ein Eindringen von Feuchtigkeit zu dem mindestens einen Anschluss, gegenüber einem nicht-abgeschirmten Zustand erheblich verlangsamt ist, beispielsweise um mindestens einen Faktor 10, vorzugsweise um mindestens einen Faktor 100 und besonders bevorzugt um mindestens einen Faktor 1000. Das Behältergehäuse kann, wie oben ausgeführt, insbesondere zumindest teilweise aus einem Kunststoff und/oder aus einem metallischen Material hergestellt sein. Das Behältergehäuse kann auch vollständig oder teilweise elektrisch abschirmende Eigenschaften aufweisen, beispielsweise eine Abschirmung gegenüber elektromagnetischer Strahlung und/oder gegenüber starken elektrischen und/oder magnetischen Feldern. Beispielsweise kann eine Abschirmung um mindestens einen Faktor 10, vorzugsweise um mindestens einen Faktor 100 und besonders bevorzugt um mindestens einen Faktor 1000 vorgesehen sein. Die Abschirmung kann beispielsweise durch mindestens eine metallische Abschirmung in dem Behältergehäuse herbeigeführt werden, wobei beispielsweise leitende Materialien zur Abschirmung eingesetzt werden können. Auch eine weichmagnetische Abschirmung kann alternativ oder zusätzlich vorgesehen sein.

Der Schutzbehälter kann weiterhin mindestens ein Trockenmittel zur Verminderung einer Feuchtigkeit in dem Innenraum des Schutzbehälters umfassen. Hierbei kann das Trockenmittel beispielsweise als separates Element in den Innenraum und/oder in einen an den Innenraum angrenzenden Raum eingebracht sein. Alternativ oder zusätzlich kann das Trockenmittel jedoch auch ganz oder teilweise in die Behälterwand integriert sein. Als Trockenmittel kommen dabei üblicherweise eingesetzte Trockenmittel in Betracht, wie beispielsweise Silicagel oder andere Trockenmittel, wie sie beispielsweise aus der Lagerung empfindlicher Komponenten, beispielsweise Sensorkomponenten, bekannt sind.

Alternativ oder zusätzlich kann der Schutzbehälter mindestens ein keimabtötendes Mittel umfassen, insbesondere ein Biozid und/oder ein Fungizid. Dieses keimabtötende Mittel, welches grundsätzlich Keime in beliebigen Stadien der Entwicklung abtöten kann, kann beispielsweise als separates Mittel, beispielsweise als Desinfektionsmittel, in den Innenraum und/oder in einen an den Innenraum angrenzenden Raum eingebracht sein. Alternativ oder zusätzlich kann das keimabtötende Mittel auch beispielsweise auch ganz oder teilweise in den Schutzbehälter, beispielsweise das Behältergehäuse, integriert sein. So können beispielsweise keimabtötende Materialien, wie beispielsweise Silberjodid, als Füllstoffe in einem Kunststoff der Behälterwand enthalten sein.

Wie oben ausgeführt, kann das Steuerteil insbesondere in den Schutzbehälter einsteckbar sein. In diesem Fall kann, wie oben ausgeführt, der Verbinder insbesondere eingerichtet sein, um mit dem Anschluss eine Steckverbindung einzugehen. Diese Steckverbindung kann insbesondere beim Einstecken des Steuerteils in den Schutzbehälter hergestellt werden. Alternativ kann die Steckverbindung auch beispielsweise vor oder nach dem Einstecken separat hergestellt werden, beispielsweise durch separates Verbinden des Anschlusses mit dem Verbinder. Beim Herstellen der Steckverbindung kann insbesondere der Sensoranschluss eine Steckverbindung mit dem Sensorverbinder eingehen, und optional der Steuerverbinder eine Steckverbindung mit dem Steueranschluss.

Wie oben dargestellt, ist der Verbinder eingerichtet, um mit dem Anschluss eine Verbindung einzugehen und diesen mediendicht zu verschließen. Der Verbinder kann dementsprechend insbesondere mindestens eine O-Ringdichtung aufweisen. So kann beispielsweise der Sensorverbinder eine O-Ringdichtung aufweisen, und optional der Steuerverbinder ebenfalls eine O-Ringdichtung. Allgemein kann der Schutzbehälter eingerichtet sein, um die Verbindung zwischen dem Verbinder und dem Anschluss zu schmieren. Beispielsweise kann eine automatische O-Ring-Schmierung vorgesehen sein, beispielsweise um ein Austrocknen des mindestens einen optionalen O-Rings durch eine Schmierung in regelmäßigen oder unregelmäßigen Abständen zu verhindern. Zu diesem Zweck kann beispielsweise ein vorzugsweise für Elektronikkomponenten kompatibles und vorzugsweise biologisch unbedenkliches Schmiermittel eingesetzt werden. Das Schmiermittel sollte hydrophob sein, um einen Feuchtefilm im Steckerbereich unter dem O-Ring hindurch zu unterbinden.

Wie oben ausgeführt, ist der Verbinder zunächst eingerichtet, um, wenn dieser mit dem Anschluss verbunden ist, den Anschluss mediendicht zu verschließen. Optional kann zusätzlich der Verbinder eine elektrische Verbindung mit dem Anschluss eingehen. So kann beispielsweise der Sensorverbinder eine elektrische Verbindung mit Kontakten des Sensoranschlusses eingehen und optional der Steuerverbinder eine elektrische Verbindung mit einem oder mehreren Kontakten des Steueranschlusses eingehen. Dementsprechend kann der Verbinder beispielsweise ganz oder teilweise als elektrischer Stecker ausgestaltet sein, mittels dessen ein oder mehrere Kontakte des Anschlusses elektrisch kontaktierbar sind, zusätzlich zu der oben beschriebenen mediendichten Verschlussfunktion.

Der Verbinder kann insbesondere eingerichtet sein, um einen ESD-Schutz für das Steuerteil bereitzustellen. Unter einem ESD-Schutz ist dabei ein Schutz gegenüber Zerstörungen durch elektrostatische Aufladungen zu verstehen. Beispielsweise kann der Sensorverbinder eingerichtet sein, um einen ESD-Schutz an dem Sensoranschluss bereitzustellen. Alternativ oder zusätzlich kann der Steuerverbinder, welcher optional vorgesehen sein kann, eingerichtet sein, um einen ESD-Schutz an dem Steueranschluss bereitzustellen.

Der ESD-Schutz kann auf verschiedene Weisen gewährleistet werden, die dem Fachmann grundsätzlich bekannt sind. Beispielsweise kann der Anschluss mindestens zwei Kontakte aufweisen. Beispielsweise kann der Sensoranschluss mindestens zwei, vorzugsweise drei oder mehr Kontakte aufweisen. Alternativ oder zusätzlich kann der Steueranschluss einen, zwei oder mehr Kontakte aufweisen. Der Verbinder kann eingerichtet sein, um die mindestens zwei Kontakte, beispielsweise die mindestens zwei Kontakte des Sensoranschlusses und/oder die mindestens zwei Kontakte des Steueranschlusses miteinander zu verbinden, beispielsweise über mindestens eines der folgenden Elemente: mindestens einen ohmschen Widerstand; mindestens eine Diode; mindestens eine Kapazität. Beispielsweise kann der Sensoranschluss zwei oder drei Kontakte aufweisen, nämlich einen Kontakt für eine Arbeitselektrode und jeweils mindestens einen Kontakt für eine Referenzelektrode und eine Gegenelektrode. Dabei können die Referenzelektrode und die Gegenelektrode getrennt ausgebildet sein, wie auch ihre Kontakte, können jedoch auch grundsätzlich zusammengefasst sein, so dass auch deren Kontakte zusammengefasst sein können. In diesem Fall können beispielsweise der Kontakt der Gegenelektrode mit dem Kontakt der Referenzelektrode verbunden sein, beispielsweise über eines oder mehrere der genannten Elemente, und der Kontakt der Referenzelektrode und der Arbeitselektrode können miteinander verbunden sein, beispielsweise über eines oder mehrere der genannten Elemente. Auch andere ESD-Schutzschaltungen sind möglich, beispielsweise eine Erdung eines oder mehrerer Kontakte.

Wie oben dargestellt, kann der Schutzbehälter als rein passiver Schutzbehälter ausgestaltet sein. Dementsprechend kann der Schutzbehälter beispielsweise einen reinen mediendichten Verschluss des Anschlusses bereitstellen. Optional kann der Schutzbehälter, wie oben ebenfalls ausgeführt, einen ESD-Schutz für das Steuerteil bereitstellen. Optional können zusätzlich jedoch auch aktive Funktionen in den Schutzbehälter integriert sein, beispielsweise eine oder mehrere aktive elektrische und/oder logische Funktionen. Insbesondere kann der Schutzbehälter eingerichtet sein, um eine Funktionsprüfung des Steuerteils vorzunehmen. Zu diesem Zweck kann der Schutzbehälter beispielsweise eingerichtet sein, um den Anschluss und insbesondere den Sensoranschluss mit mindestens einem Prüfstrom und/oder mindestens einer Prüfspannung zu beaufschlagen. Beispielsweise kann der Schutzbehälter dann eingerichtet sein, um mindestens ein von dem Steuerteil generiertes Signal, insbesondere ein Messsignal und/oder ein Messergebnis, abzufragen. Dazu versorgt vorzugsweise der Schutzbehälter das Steuerteil mit einer Versorgungsspannung, beispielsweise über den Steueranschluss. Diese Abfrage kann insbesondere drahtlos vorgenommen werden, beispielsweise über einen Telemetriebaustein des Steuerteils, also einen Baustein, welcher für eine drahtlose Kommunikation (beispielsweise eine Kommunikation über Funk) zwischen dem Steuerteil und einem anderen Gerät, beispielsweise einem Datenmanager, ausgestaltet ist. Der Schutzbehälter kann insbesondere eingerichtet sein, um mindestens ein Ergebnis der Funktionsprüfung an mindestens ein weiteres Gerät und/oder mindestens einen Benutzer zu kommunizieren. Das mindestens eine weitere Gerät kann beispielsweise eines oder mehrere der folgenden Geräte umfassen: einen mit dem Schutzbehälter über mindestens eine drahtgebundene oder drahtlose Verbindung verbundenen Datenmanager zur Verwaltung von Messergebnissen des transkutanen Sensorsystems; einen mit dem Schutzbehälter über eine drahtgebundene oder drahtlose Verbindung verbundenen Computer, insbesondere einen Arztcomputer und/oder einen Patientencomputer; ein Computernetzwerk; ein mobiles Kommunikationsgerät wie beispielsweise ein Mobiltelefon oder einen PDA (persönlicher digitaler Assistent, Personal Digital Assistant). Auch weitere Geräte sind, alternativ oder zusätzlich, einsetzbar.

Der Schutzbehälter kann eingerichtet sein, um einem Benutzer ein Ergebnis der Funktionsprüfung über mindestens eine Benutzerschnittstelle zu übermitteln. Diese Benutzerschnittstelle kann insbesondere mindestens ein Anzeigenelement umfassen. Dieses Anzeigenelement kann beispielsweise ein Display, eine Leuchtanzeige, eine akustische Anzeige, eine haptische Anzeige oder Kombinationen der genannten und/oder anderer Anzeigenelemente umfassen.

Die Funktionsprüfung kann beispielsweise eine Datensammlung umfassen. Beispielsweise können in regelmäßigen Abständen Funktionsprüfungen durchgeführt werden und in dem Schutzbehälter gespeichert werden. Die Ergebnisse der Funktionsprüfungen können, wie oben dargestellt und wie unten noch näher ausgeführt wird, beispielsweise an ein anderes Gerät und/oder an einen Benutzer und/oder auf andere Weise kommuniziert werden.

Der Schutzbehälter kann insbesondere eingerichtet sein, um bei der Funktionsprüfung eine Leckstrommessung zwischen mindestens zwei Kontakten des Anschlusses, insbesondere zwischen mindestens zwei Kontakten des Sensoranschlusses und/oder des Steueranschlusses, vorzunehmen. Überschreitet beispielsweise ein gemessener Leckstrom eine oder mehrere Toleranzschwellen, so kann auf einen technischen Defekt und/oder eine Verschmutzung geschlossen werden. Bei einem Leckstrom handelt es sich um einen Strom zwischen mindestens zwei Kontakten oder Prüfpunkten, zwischen welchen idealerweise ein sehr hoher Widerstand (>10¹² Ohm) liegen sollte.

Der Schutzbehälter kann insbesondere eingerichtet sein, wie oben ausgeführt, um mindestens ein von dem Steuerteil generiertes Signal, insbesondere ein Messsignal und/oder Messergebnis, abzufragen. Diese Abfrage kann insbesondere während der Funktionsprüfung erfolgen und/oder beispielsweise Bestandteil der Funktionsprüfung sein, beispielsweise während der genannten Leckstrommessung. Auch während einer Beaufschlagung mit dem mindestens einen Prüfstrom kann eine derartige Abfrage alternativ oder zusätzlich erfolgen.

Der Schutzbehälter kann insbesondere eingerichtet sein, um mindestens ein Ergebnis der Funktionsprüfung an mindestens ein weiteres Gerät und/oder einen Benutzer zu übermitteln. Diese Übermittlung kann beispielsweise dadurch erfolgen, dass der Schutzbehälter mindestens eine Schnittstelle umfasst. Beispielsweise kann eine Benutzerschnittstelle vorgesehen sein, um dem Benutzer das Ergebnis der Funktionsprüfung mitzuteilen. Diese Benutzerschnittstelle kann beispielsweise mindestens ein Anzeigenelement, beispielsweise mindestens ein Display und/oder eine Leuchtanzeige und/oder eine andere Art visueller Anzeige und/oder eine akustische Anzeige und/oder ein haptisches Anzeigenelement umfassen. Alternativ oder zusätzlich kann die Schnittstelle zu einem Benutzer und/oder einem anderen Gerät eine oder mehrere der folgenden Schnittstellen umfassen: eine unidirektionale Datenschnittstelle; eine bidirektionale Datenschnittstelle; ein Anzeigenelement zur visuellen Anzeige mindestens einer Information, insbesondere ein Display und/oder eine Leuchtanzeige; ein Anzeigenelement zur akustischen Ausgabe mindestens einer Information; ein Anzeigenelement zur haptischen Ausgabe mindestens einer Information; mindestens ein Bedienelement zur Eingabe einer Information und/oder eines Steuerbefehls, beispielsweise mindestens eine Eingabetaste. Die mindestens eine optionale Datenschnittstelle kann beispielsweise eine drahtgebundene oder drahtlose Datenschnittstelle umfassen. Beispielsweise können Infrarot-Datenschnittstellen, USB-Datenschnittstellen, serielle Schnittstellen oder andere Arten von Schnittstellen, wie sie dem Fachmann aus dem Stand der Technik grundsätzlich bekannt sind, zum Einsatz kommen.

Der Schutzbehälter kann weiterhin eingerichtet sein, um Daten der Funktionsprüfung zu speichern. Zu diesem Zweck kann der Schutzbehälter beispielsweise mindestens ein Speicherelement umfassen. Weiterhin kann der Schutzbehälter, alternativ oder zusätzlich, mindestens eine eigene Intelligenz umfassen. Dementsprechend kann der Schutzbehälter beispielsweise ein Datenverarbeitungsgerät aufweisen, insbesondere mindestens einen Microcontroller, welcher gegebenenfalls zusätzlich Ein- und Ausgabeelemente sowie optional einen oder mehrere Speicher umfassen kann. Das Datenverarbeitungsgerät kann beispielsweise die oben beschriebene Funktionsprüfung in einer oder mehreren der beschriebenen Ausgestaltungen steuern oder zumindest unterstützen.

Der Schutzbehälter kann weiterhin eingerichtet sein, um eine Warnung an einen Benutzer und/oder ein weiteres Gerät zu übermitteln, wenn bei der Funktionsprüfung eine Fehlfunktion des Steuerteils ermittelt wird. Unter einer Warnung ist dabei grundsätzlich eine beliebige Information über eine Fehlfunktion zu verstehen, welche beispielsweise in einem Warnsignal und/oder auf eine andere Weise gestalteten Warninformation bestehen kann, beispielsweise einem Fehlerbit bei der Übermittlung von Daten. Unter eine Fehlfunktion ist beispielsweise eine Abweichung von Messergebnissen der Funktionsprüfung von vorgegebenen Normbereichen und/oder Normwerten zu verstehen, beispielsweise ein Überschreiten und/oder Unterschreiten bestimmter Schwellwerte.

Der Schutzbehälter kann insbesondere mindestens eine elektrische Energieversorgung aufweisen. Diese elektrische Energieversorgung kann beispielsweise genutzt werden, um auch das in den Schutzbehälter eingebrachte Steuerteil mit elektrischer Energie zu versorgen. Alternativ oder zusätzlich kann die Energieversorgung auch beispielsweise eigene Komponenten des Schutzbehälters versorgen, beispielsweise das genannte optionale Datenverarbeitungsgerät und/oder die genannte optionale Schnittstelle. Die elektrische Energieversorgung kann insbesondere eine oder mehrere der folgenden Energieversorgungen umfassen: eine interne Energieversorgung, insbesondere mindestens einen internen Energiespeicher, insbesondere mindestens eine Batterie und/oder mindestens einen Akkumulator; einen Anschluss für eine externe Energieversorgung, insbesondere einen Netzstecker und/oder einen Netzanschluss.

Der Schutzbehälter kann weiterhin auch in das Messkonzept des transkutanen Sensorsystems eingebunden sein. So kann der Schutzbehälter beispielsweise eingerichtet sein, um mindestens eine Kalibrationsinformation zu empfangen, insbesondere eine Kalibrationsinformation von einem externen Messgerät zum Nachweis des mindestens einen Analyten mittels mindestens eines Testelements. Alternativ kann jedoch auch ein derartiges Messgeräte zum Nachweis des mindestens einen Analyten mittels mindestens eines Testelements in den Schutzbehälter selbst integriert sein. Der Schutzbehälter kann eingerichtet sein, um die Kalibrationsinformation an das Steuerteil weiterzugeben, beispielsweise über den mindestens einen Anschluss. Ausführungsbeispiele werden unten noch näher erläutert.

Neben dem Schutzbehälter in einer oder mehreren der oben beschriebenen Ausgestaltungen wird weiterhin ein Kit vorgeschlagen. Dieses Kit umfasst mindestens einen Schutzbehälter in einer oder mehreren der oben beschriebenen Ausgestaltungen und weiterhin mindestens ein wieder verwendbares Steuerteil eines transkutanen Sensorsystems zum Nachweis mindestens eines Analyten in einer Körperflüssigkeit. Das Steuerteil kann beispielsweise den oben dargestellten Ausgestaltungen, welche das Steuerteil betreffen, entsprechen. Das Steuerteil umfasst dementsprechend mindestens einen Anschluss, welcher wiederum mindestens einen Sensoranschluss zur Verbindung mit mindestens einem transkutanen Sensor sowie optional mindestens einen Steueranschluss zur Verbindung des Steuerteils mit mindestens einer Steuerkomponente, insbesondere mindestens einer Einweg-Steuerkomponente, aufweist.

Das Steuerteil kann weiterhin mindestens eines der folgenden Elemente umfassen: ein Datenverarbeitungsgerät, insbesondere einen Microcontroller; einen Datenspeicher; eine Auswerteelektronik zum Ansteuern und/oder Auswerten des mindestens einen transkutanen Sensors; eine unidirektionale und/oder bidirektionale Schnittstelle zum Austausch von Daten, insbesondere Messdaten, mit mindestens einem weiteren Gerät, beispielsweise mit mindestens einem Datenmanager, insbesondere drahtlose Schnittstelle, insbesondere einen Telemetriebaustein. Bezüglich weiterer möglicher Ausgestaltungen des wieder verwendbaren Steuerelements kann beispielsweise auf den oben zitierten Stand der Technik verwiesen werden, insbesondere auf US 2008/0242962 A1. Das Steuerteil kann insbesondere der dort beschriebenen wieder verwendbaren Basisstation (base station) entsprechen. Auch andere Ausgestaltungen sind jedoch grundsätzlich möglich. Das Steuerteil kann insbesondere eingerichtet sein, um über den Anschluss, insbesondere den Steueranschluss, mit elektrischer Energie versorgt zu werden, beispielsweise mit elektrischer Energie aus einer Energieversorgung des Schutzbehälters, beispielsweise einem Energiespeicher des Schutzbehälters. Dementsprechend kann das Steuerteil beispielsweise über keine eigene interne Energieversorgung, insbesondere über keinen eigenen Energiespeicher, verfügen.

In einem weiteren Aspekt der vorliegenden Erfindung wird eine Sensorvorrichtung zum Nachweis mindestens eines Analyten in einer Körperflüssigkeit vorgeschlagen. Diese Sensorvorrichtung umfasst mindestens einen transkutanen Sensor, wobei der transkutane Sensor zumindest teilweise in ein Körpergewebe implantierbar ist. Unter einer Implantierbarkeit ist somit eine Eigenschaft zu verstehen, bei welcher mindestens ein Teil des transkutanen Sensors in ein Körpergewebe, beispielsweise ein interstitielles Gewebe, eingebracht werden kann. Andere Bereiche des Sensors können dabei aus dem Körpergewebe herausragen und beispielsweise durch eine Haut des Benutzers hindurch nach außen geführt sein. Die Implantierbarkeit ist beispielsweise dadurch gewährleistet, dass biokompatible Materialien für den zu implantierenden Bereich des transkutanen Sensors verwendet werden, beispielsweise Materialien, welche selbst über biokompatible Eigenschaften verfügen oder welche mit biokompatiblen Materialien beschichtet sind, so dass ein Eindringen von nicht-biokompatiblen Materialien und/oder ein direkter Kontakt von nicht-biokompatiblen Materialien mit dem Körpergewebe und/oder Körperflüssigkeiten verhindert werden kann.

Der transkutane Sensor kann grundsätzlich auf einem beliebigen Sensorprinzip zum Nachweis des mindestens einen Analyten basieren. Beispielsweise kann der Sensor zu diesem Zweck mindestens ein Sensormaterial aufweisen, welches bei Anwesenheit des mindestens einen nachzuweisenden Analyten spezifisch mindestens eine physikalische und/oder chemisch nachweisbare Eigenschaft ändert. Unter "spezifisch" ist dabei zu verstehen, dass diese Eigenschaft vorzugsweise ausschließlich bei Anwesenheit des nachzuweisenden Analyten und gegebenenfalls chemisch sehr ähnlicher Analyte auftritt, nicht hingegen bei Anwesenheit anderer Materialien oder lediglich bei Anwesenheit von Materialien, deren Auftreten in der Körperflüssigkeit sehr unwahrscheinlich ist. Die mindestens eine nachzuweisende Eigenschaft kann beispielsweise, wie oben ausgeführt, eine elektrochemische Eigenschaft sein. Dementsprechend kann der Sensor beispielsweise ein elektrochemischer Sensor sein oder einen elektrochemischen Sensor umfassen. Zu diesem Zweck kann der Sensor beispielsweise mindestens eine Arbeitselektrode und mindestens eine Gegenelektrode und/oder mindestens eine Referenzelektrode umfassen. Die mindestens eine Arbeitselektrode kann beispielsweise mit mindestens einer Nachweischemie beschichtet sein, beispielsweise mindestens einer Nachweischemie, welche mindestens ein Enzym und/oder mindestens einen Mediator umfasst. Beispielsweise kann das Enzym Glucoseoxidase oder Glucosedehydrogenase umfassen. Derartige transkutane Sensorsysteme sind dem Fachmann grundsätzlich bekannt. Der Nachweis des mindestens einen Analyten kann beispielsweise mittels einer bekannten potentiostatischen Messung erfolgen, mittels derer beispielsweise das Elektrodenpotential der Gegenelektrode gegenüber der Arbeitselektrode geregelt wird, wobei das Potenzial der Referenzelektrode der Regelbezug ist. Der durch die Arbeitselektrode proportional zur Glucosekonzentration oder allgemein zur Analytkonzentration generierte Strom kann als Messgröße ausgewertet werden.

Die Sensorvorrichtung umfasst weiterhin ein Kit gemäß der obigen Beschreibung, also ein Kit in einer oder mehreren der oben beschriebenen Ausgestaltungen, also mindestens ein wieder verwendbares Steuerteil und mindestens einen Schutzbehälter.

Die Sensorvorrichtung kann weiterhin mindestens eine Steuerkomponente umfassen. Diese Steuerkomponente kann insbesondere als Einweg-Steuerkomponente ausgestaltet sein oder mindestens eine Einweg-Steuerkomponente umfassen. Grundsätzlich ist jedoch, alternativ oder zusätzlich, auch eine Ausgestaltung als Mehrweg-Steuerkomponente möglich. Die Steuerkomponente kann beispielsweise mit dem Sensor mechanisch und/oder elektrisch verbindbar sein. Die Steuerkomponente kann beispielsweise eines oder mehrere der folgenden Bauelemente umfassen: eine elektrische Energieversorgung, insbesondere einen integrierten elektrischen Energiespeicher, vorzugsweise mindestens eine Batterie und/oder mindestens einen Akkumulator; ein Datenspeicherelement, insbesondere ein nichtflüchtiges Datenspeicherelement, beispielsweise ein EEPROM und/oder ein Flash-EPROM. Die Steuerkomponente kann beispielsweise gemäß dem oben beschriebenen Stand der Technik ausgestaltet sein. So kann die Steuerkomponente beispielsweise gemäß der Sensorträgereinheit (sensor carrier unit) in US 2008/0242962 A1 ausgestaltet sein, so dass bezüglich möglicher Details dieser Steuerkomponente auf diese Druckschrift verwiesen werden kann. Auch andere Ausgestaltungen sind jedoch möglich.

Begrifflich ist somit im Rahmen der vorliegenden Erfindung zwischen dem eigentlichen transkutanen Sensor, dem Steuerteil, der Steuerkomponente, dem Schutzbehälter, der Sensorvorrichtung und dem transkutanen Sensorsystem zu unterscheiden. Diese Elemente können insbesondere einzeln oder insgesamt als unabhängig voneinander handhabbare Elemente ausgestaltet sein, insbesondere als getrennt voneinander ausgebildete Elemente, welche beispielsweise getrennt hergestellt, gelagert, verpackt und transportiert werden können. Ein transkutanes Sensorsystem stellt eine Einheit dar, mittels derer tatsächlich die Messungen durchgeführt werden können. Das transkutane Sensorsystem umfasst somit den transkutanen Sensor, das Steuerteil und optional die Steuerkomponente. Beispielsweise kann, wie oben beschrieben, das wieder verwendbare Steuerteil im Betrieb des transkutanen Sensorsystems über den Sensoranschluss mit dem transkutanen Sensor und über den Steueranschluss mit der Steuerkomponente verbunden oder verbindbar sein. Alle drei Komponenten bilden gemeinsam somit eine Einheit, welche als transkutanes Sensorsystem bezeichnet wird. Von diesem transkutanen Sensorsystem ist begrifflich die oben beschriebene Sensorvorrichtung zu unterscheiden, welche das Kit mit dem Schutzbehälter und dem Steuerteil umfasst sowie weiterhin mindestens einen transkutanen Sensor und optional die mit dem Sensor verbindbare Steuerkomponente.

In einem weiteren Aspekt der vorliegenden Erfindung wird ein Verfahren zum Schutz eines wieder verwendbaren Steuerteils eines transkutanen Sensorsystems zum Nachweis mindestens eines Analyten in einer Körperflüssigkeit vorgeschlagen. Das Steuerteil umfasst mindestens einen Anschluss mit mindestens einem Sensoranschluss zur Verbindung mit mindestens einem transkutanen Sensor, sowie optional mindestens einen Steueranschluss zur Verbindung des Steuerteils mit mindestens einer Steuerkomponente, insbesondere mindestens einer Einweg-Steuerkomponente. Bei dem Verfahren wird das Steuerteil von dem transkutanen Sensor getrennt und gegenüber Umwelteinflüssen abgeschirmt. Dabei wird der Anschluss mediendicht verschlossen. Bezüglich der Möglichkeiten des Verschlusses, welcher einen reinen mechanischen Verschluss zum Schutz vor einem Eindringen von Umwelteinflüssen, wie beispielsweise Staub oder Feuchtigkeit, bereitstellen kann, kann beispielsweise optional ein Verschluss unter Bereitstellung eines ESD-Schutzes erfolgen. Wiederum alternativ oder zusätzlich kann auch bei dem Verschluss beispielsweise eine Funktionsprüfung in einer der oben beschriebenen Ausgestaltungen erfolgen. Das Verschließen kann insbesondere unter Verwendung eines Schutzbehälters in einer oder mehreren der oben beschriebenen Ausgestaltungen erfolgen, so dass bezüglich der Möglichkeiten des Verschlusses beispielsweise auf die obige Beschreibung verwiesen werden kann.

Die oben beschriebenen Vorrichtungen und Verfahren weisen gegenüber bekannten Vorrichtungen und Verfahren zahlreiche Vorteile auf. Insbesondere können Funktionsausfälle, welche durch Verschmutzungen eines wieder verwendbaren Steuerteils entstehen, wirksam vermieden oder zumindest reduziert werden. Der Schutzbehälter kann als tragbarer Schutzbehälter ausgestaltet sein und kann von einem Benutzer beispielsweise in einer Manteltasche mitgeführt werden oder Bestandteil eines tragbaren Satzes von Zubehörteilen sein. Durch die beschriebene Möglichkeit der Einbindung des Schutzbehälters in Funktionsprüfungen kann der Schutzbehälter Bestandteil eines gesamten Failsafe-Konzepts sein und kann somit wirksam zur Steigerung der Benutzerfreundlichkeit und zur Steigerung der Zuverlässigkeit der Sensorvorrichtung und des transkutanen Sensorsystems beitragen.

### Kurze Beschreibung der Figuren

Weitere Einzelheiten und Merkmale der Erfindung ergeben sich aus der nachfolgenden Beschreibung bevorzugter Ausführungsbeispiele. Die Ausführungsbeispiele sind in den Figuren schematisch dargestellt. Gleiche Bezugszeichen in den einzelnen Figuren bezeichnen dabei gleiche oder funktionsgleiche bzw. hinsichtlich ihrer Funktionen einander entsprechende Elemente. Die Erfindung ist nicht auf die Ausführungsbeispiele beschränkt.

Im Einzelnen zeigen:
Figur 1 eine schematische Darstellung eines transkutanen Sensorsystems;
Figur 2 eine perspektivische Darstellung eines Disposable-Teils eines transkutanen Sensorsystems;
Figur 3 eine perspektivische Darstellung eines Reusables eines transkutanen Sensorsystems;
Figur 4 zwei Ausführungsbeispiele transkutaner Sensorsysteme in perspektivischer Darstellung;
Figur 5 ein transkutanes Sensorsystem mit abgenommenem Deckel;
Figur 6 ein Ausführungsbeispiel eines Kits mit einem Schutzbehälter und einem wieder verwendbaren Steuerteil;
Figur 7 eine schematische Ausschnittsdarstellung einer Bereitstellung eines ESD-Schutzes;
Figur 8 ein zu Figur 6 alternatives Ausführungsbeispiel eines Kits;
Figur 9 einen Ablaufplan einer Failsafe-Routine;
Figur 10 ein erstes Ausführungsbeispiel eines Verfahrens zum Schutz eines wieder verwendbaren Steuerteils eines transkutanen Sensorsystems; und
Figur 11 ein zweites Ausführungsbeispiel eines Verfahrens zum Schutz eines wieder verwendbaren Steuerteils eines transkutanen Sensorsystems.

### Ausführungsbeispiele

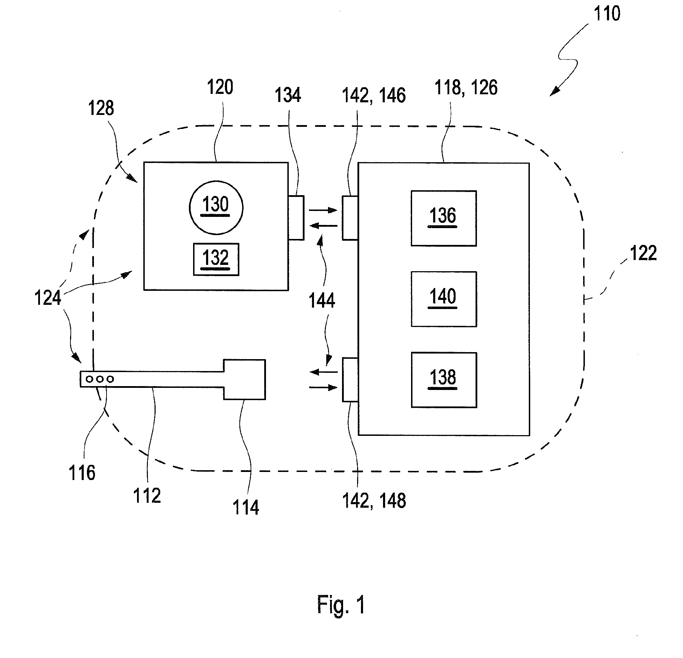

In Figur 1 ist exemplarisch ein Ausführungsbeispiel eines transkutanen Sensorsystems 110 zum Nachweis mindestens eines Analyten in einer Körperflüssigkeit schematisch dargestellt. Das transkutane Sensorsystem 110 in dem dargestellten Ausführungsbeispiel kann beispielsweise dem in US 2008/0242962 A1 beschriebenen transkutanen Sensorsystem entsprechen. Das transkutane Sensorsystem 110 umfasst in dem dargestellten Ausführungsbeispiel einen transkutanen Sensor 112 mit einem Sensorstecker 114 und einem in das Körpergewebe einbringbaren Sensorbereich 116. Weiterhin umfasst das transkutane Sensorsystem 110 ein wieder verwendbares Steuerteil 118 und eine Steuerkomponente 120, insbesondere eine Einweg-Steuerkomponente. Die Einweg-Steuerkomponente kann insbesondere für einen einmaligen Gebrauch, beispielsweise für lediglich eine einzige Messperiode, ausgestaltet sein. Auch eine andere Ausgestaltung ist jedoch möglich. Die Steuerkomponente 120, der transkutane Sensor 112 und ein Befestigungselement 122, beispielsweise in Form eines Pflasters zum Aufkleben auf eine Hautoberfläche, sowie gegebenenfalls weitere Komponenten wie beispielsweise Gehäusekomponenten, können gemeinsam ein so genanntes Diposable 124, also ein Einwegteil, bilden, wohingegen das wieder verwendbare Steuerteil ein so genanntes Reusable, also ein Mehrweg-Teil, bildet. Die Steuerkomponente 120 wird häufig auch als Bodymount 128 bezeichnet.

Die Steuerkomponente 120 umfasst in dem dargestellten Ausführungsbeispiel exemplarisch einen elektrischen Energiespeicher 130 und/oder einen Datenspeicher 132. Der Datenspeicher 132 kann beispielsweise als nicht-flüchtiger Datenspeicher ausgestaltet sein, beispielsweise als ROM und/oder EEPROM und/oder Flash- EPROM. Weiterhin umfasst die Steuerkomponente 120 in dem dargestellten Ausführungsbeispiel einen Steuerstecker 134.

Das wieder verwendbare Steuerteil 118 umfasst in dem dargestellten Ausführungsbeispiel seinerseits eine Datenverarbeitungsvorrichtung in Form eines Microcontrollers 136, eine Ansteuer- und Auswerteelektronik 138 zum Ansteuern des transkutanen Sensors 112 sowie einen Telemetriebaustein 140 zum drahtlosen Kommunizieren von Messergebnissen, beispielsweise an einen in Figur 1 nicht dargestellten Datenmanager, welcher ebenfalls optional Bestandteil des transkutanen Sensorsystems 110 sein kann. Weiterhin kann das wieder verwendbare Steuerteil 118 einen oder mehrere Speicher umfassen, welche auch als flüchtige Speicher ausgestaltet sein können und/oder auch als nicht-flüchtige Speicher. Während der Datenspeicher 132 in der Steuerkomponente 120 vorzugsweise zur permanenten Speicherung von Chargeninformationen über das Sensorelement 112 dient, beispielsweise von herstellerspezifischen und/oder herstellungsspezifischen Informationen, wie beispielsweise Kalibrationsinformationen, kann der Datenspeicher des wieder verwendbaren Steuerteils 118, welcher beispielsweise Bestandteil des Microcontrollers 136 sein kann, zum Speichern von Messergebnissen dienen, welche anschließen über den Telemetriebaustein 140 an den Datenmanager weitergegeben werden können.

Entsprechend kann das wieder verwendbare Steuerteil 118 mindestens einen Anschluss 142 umfassen, welcher in diesem Ausführungsbeispiel exemplarisch zweiteilig ausgestaltet ist und eine Steckverbindung 144 oder eine andere Art von Verbindung eingehen kann. Der Anschluss 142 umfasst in dem dargestellten Ausführungsbeispiel einen Steueranschluss 146 zum Verbinden mit dem Steuerstecker 134 und einen Sensoranschluss 148 zum Verbinden mit dem Sensorstecker 114. Auch andere Ausgestaltungen sind jedoch grundsätzlich möglich.

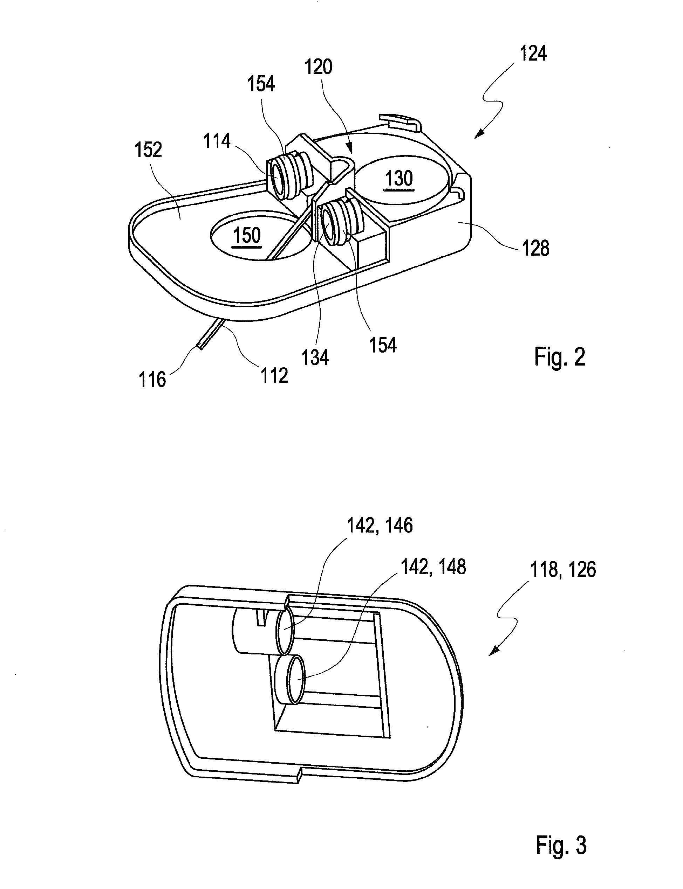

In den Figuren 2 bis 5 sind verschiedene perspektivische Darstellungen von Komponenten des transkutanen Sensorsystems 110 gemäß Figur 1 gezeigt. Diese sollen im Folgenden gemeinsam erläutert werden.

So zeigt Figur 2 eine perspektivische Darstellung eines Bodymounts 128 ohne das Befestigungselement 122, mit montiertem transkutanen Sensor 112. Hieraus ist erkennbar, dass der transkutane Sensor 112 durch ein Loch 150 in einer Bodenplatte 152 geführt werden kann, so dass der Sensorbereich 116 in eine Körpergewebe eines Benutzers hineinragt. Weiterhin ist aus dieser perspektivischen Darstellung erkennbar, dass der Sensorstecker 114 und der Steuerstecker 134 jeweils optional Dichtelemente 154 umfassen können, beispielsweise O-Ringe.

In Figur 3 ist eine perspektivische Ansicht eines Ausführungsbeispiels des wieder verwendbaren Steuerteils 118 gezeigt, in einer Ansicht von unten. Der Steueranschluss 146 und der Sensoranschluss 148 sind in dieser Darstellung erkennbar. Zum Herstellen der Steckverbindung 144 in Figur 1 können beispielsweise aktive Steckelemente, beispielsweise federnde Elemente, ausschließlich auf der Seite des Reusables 126, also des Steuerteils 118, angeordnet sein.

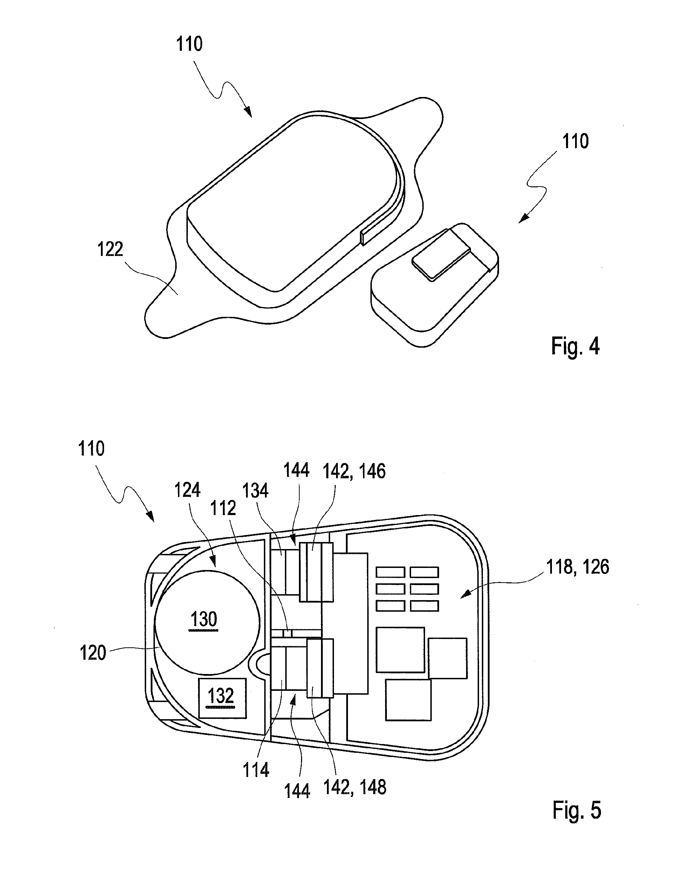

In Figur 4 ist das fertige transkutane Sensorsystem 110 nochmals in zwei verschiedenen Ausführungsbeispielen dargestellt. In Figur 5 ist nochmals das komplette transkutane Sensorsystem 110 mit geöffnetem Gehäuse dargestellt. Dabei sind die Steuerkomponente 120, das wieder verwendbare Steuerteil 118 und der Anschluss 142 im verbundenen Zustand deutlich erkennbar. Das Disposable 124 kann alle Teile umfassen, die nur für einen Messzyklus verwendet werden, also den Sensor 112, das Bodymount 128 ohne das Reusable 126, und gegebenenfalls eine Insertionsnadel, die in Figur 5 nicht dargestellt ist. Die gesamte funktionale Einheit des transkutanen Sensorsystems 110 mit Sensor 112, Bodymount 128 und Reusable 126 wird häufig auch als "Patch" bezeichnet.

Die passiven Teile der Steckverbindung 144 in Figur 5, beispielsweise Kontaktleiterbahnen, können sich vollständig im Bodymount 128 befinden. Der Teil des Disposables 124 hin bis zur Steckverbindung 144 ist in Figur 5 geöffnet dargestellt, also der Teil links der Steckverbindung 144 in Figur 5. Dieser Teil kann hermetisch abgedichtet sein, beispielsweise durch einen Lack, eine Versiegelung und/oder einen Verguss. Damit wird die im Reusable 126 gegebene hermetische galvanische Isolation des elektrischen Kreises fortgesetzt. Durch die Dichtelemente 154, beispielsweise die O-Ringe, kann gewährleistet werden, dass das transkutane Sensorsystem 110 im fehlerfreien Betriebsfall einen elektrischen Strom nur noch zwischen den Elektroden des Sensorbereichs 116, und dort nur noch zwischen einer Arbeitselektrode und einer Gegenelektrode, zulässt.

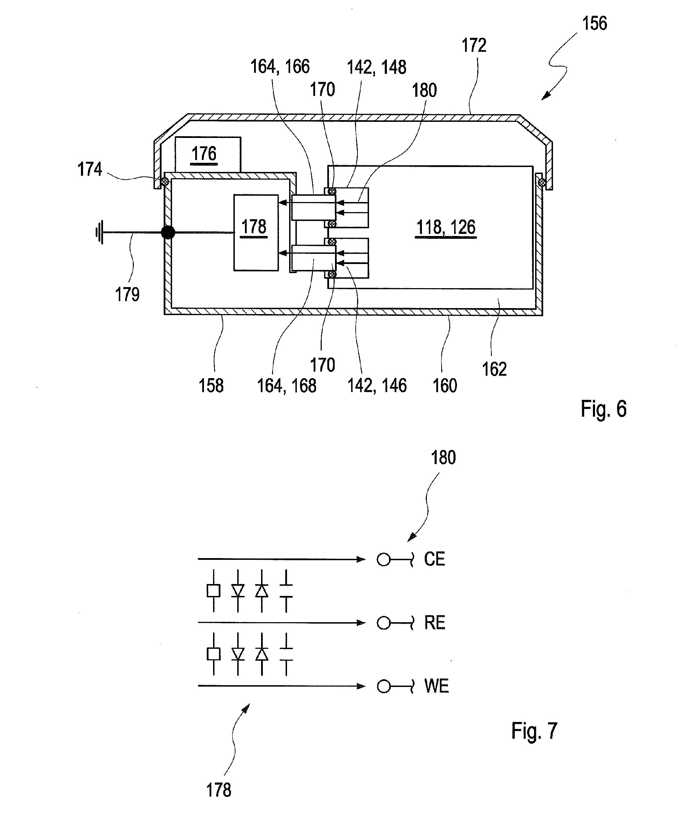

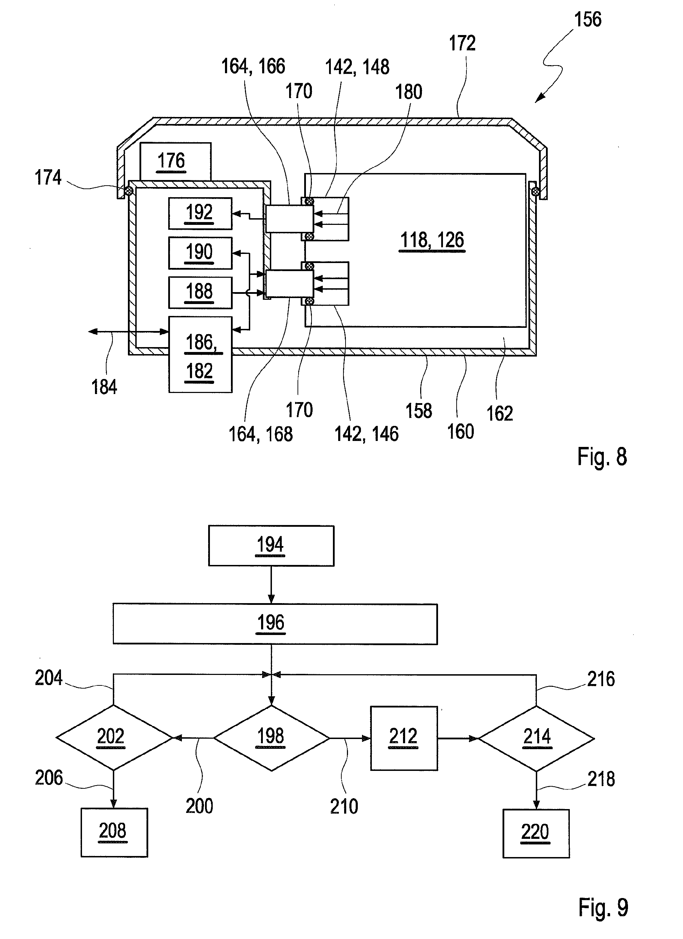

In den Figuren 6 und 8 sind verschiedene Ausführungsbeispiele eines erfindungsgemäßen Kits 156 dargestellt, umfassend das wieder verwendbare Steuerteil 118 in Form des Reusables 126 und einen Schutzbehälter 158. Der Schutzbehälter 158 kann beispielsweise als Box oder Aufbewahrungsbehälter ausgestaltet sein und umfasst ein Behältergehäuse 160 mit einem Innenraum 162, in welchem das wieder verwendbare Steuerteil 118 aufnehmbar ist.

Weiterhin umfasst der Schutzbehälter 158 jeweils mindestens einen Verbinder 164 zur Verbindung mit dem Anschluss 142. Der Verbinder 164 umfasst dabei in dem dargestellten Ausführungsbeispiel einen Sensorverbinder 166 zum Herstellen einer Verbindung mit dem Sensoranschluss 148, und optional einen Steuerverbinder 168 zum Verbinden mit dem Steueranschluss 146 des Reusables 126. Die Verbinder 166, 168 können beispielsweise als Verschlussstopfen ausgestaltet sein und können mit einem oder mehreren Dichtelementen 170 ausgestaltet sein, beispielsweise mit O-Ringen. Auf diese oder auf eine andere Weise kann der Verbinder 164 den Anschluss 142 mediendicht verschließen. Weiterhin kann der Schutzbehälter 158 ein Verschlusselement 172 aufweisen, beispielsweise einen Box-Deckel oder eine andere Art von Verschlusselement, wobei dieses Verschlusselement 172 ein oder mehrere zusätzliche Dichtelemente 174 aufweisen kann, beispielsweise eine Box-Dichtung, welche den Innenraum 162 innerhalb des Behältergehäuses 160, beispielsweise innerhalb der Box-Chassis, gegenüber Umwelteinflüssen abdichten können, vorzugsweise gegenüber Luftfeuchtigkeit, Staub, Verschmutzungen oder ähnlichen Umwelteinflüssen. Weiterhin kann das Behältergehäuse 160 eine magnetische, eine elektromagnetische oder eine elektrische Abschirmung bereitstellen. Zusätzlich kann in dem Behältergehäuse 160 mindestens ein Trockenmittel 176 aufgenommen sein, um die Feuchtigkeit innerhalb des Innenraums 162 zusätzlich zu verringern.

Der Schutzbehälter 158 kann für das Reusable 126 während Lagerzeiten zwischen den Einzelintervallen mit verschiedenen zusätzlichen Funktionalitäten ausgestaltet sein. Der Schutzbehälter 158 kann dabei verschiedenen Einsatzbedingungen gerecht werden. So können beispielsweise folgende Einsatzbedingungen vorgesehen sein:
- Einzelbenutzer, Permanent-Überwachung: Bei dieser möglichen Einsatzbedingung werden mittels eines Reusables 126 bis zu vorzugsweise 52 transkutane Sensoren 112 nacheinander, quasi-permanent von ein und demselben Benutzer eingesetzt und getragen.
- Einzelbenutzer, sporadischer Einsatz: In diesem Fall setzt der Benutzer ein Reusable 126 nur für wenige transkutane Sensoren 112 an sich selbst ein. Zwischen den Einsätzen können Wochen bis Monate liegen. Die Verwendung soll aus hygienischen Gründen nicht länger als 1 Jahr betragen.
- mehrere Benutzer: In einer Klinik oder einer sonstigen Einrichtung wird von einem Fachpersonal ein Reusable 126 an unterschiedlichen Benutzern angelegt. Die Einsatzfrequenz kann selten bis häufig sein, wobei jedoch 52 Einsätze und/oder eine Benutzungsdauer von 1 Jahr nicht überschritten werden sollte.

Weiter Einsatzszenarien sind denkbar. Unabhängig von dem gewählten Einsatzszenario ist in den meisten Fällen eine wesentliche Anforderung an den Schutzbehälter 158, den Anschluss 142 auf der Seite des Reusables 126 vor einem Eindringen von Staub, Salzen und Feuchtigkeit zu schützen. Der kritischste Fall bezüglich einer Feuchtigkeit ist das Eindringen von Wasserdampf. Ein Eindringen von Tropf- und Schwallwasser ist hingegen in den meisten Fällen in einer Lagerphase bestimmungsgemäß eher nicht zu erwarten.

Ferner kann der Schutzbehälter 158 eingerichtet sein, um eine elektrostatische Aufladung (ESD) und dadurch mögliche Beschädigung während der Lagerung und/oder der Entnahme zu vermeiden oder zumindest zu reduzieren. Dies ist in Figur 6 exemplarisch dargestellt. Dort weist der Schutzbehälter 158 beispielsweise lediglich passive Funktionen auf. So kann neben der Trockenhaltung des Innenraums 162 und/oder des Anschlusses 142 alternativ oder zusätzlich ein Kontaktschutz, eine biozide und/oder fungizide Funktion, ein Schutz gegen elektrische Aufladung, beispielsweise in Form einer leitenden Beschichtung von Behältergehäuse 160 und/oder Verschlusselement 172, oder Kombinationen der genannten und/oder anderer Funktionen gegeben sein. Die biozide und/oder fungizide Funktion kann beispielsweise durch Verwendung mindestens eines Biozids und/oder Fungizids realisiert werden, welche auch beispielsweise ganz oder teilweise in das Behältergehäuse 160 eingebracht und/oder integriert sein können. Die Biozide und/oder Fungizide sollten dabei keine Beeinträchtigung der so genannten Biokompatibilität bewirken. Weiterhin kann ein ESD-Schutz 178 für den Anschluss 172 vorgesehen sein, also für den Steueranschluss 146 und/oder den Sensoranschluss 148. Beispielsweise können der Steueranschluss 146 und/oder der Sensoranschluss 148 einen oder mehrere Kontakte 180 umfassen, für welche ein ESD-Schutz 178 bereitgestellt werden kann. Dies ist in Figur 7 exemplarisch für Kontakte 180 des Sensoranschlusses 148 dargestellt. Diese Kontakte 180 können beispielsweise einen Kontakt für eine Arbeitselektrode (WE), einen Kontakt für eine Referenzelektrode (RE) und einen Kontakt für eine Gegenelektrode (CE) umfassen. Dabei können die Kontakte für die Referenzelektrode und für die Gegenelektrode getrennt ausgebildet sein, können jedoch grundsätzlich auch zusammengefasst werden. Wie in Figur 7 gezeigt, kann der ESD-Schutz 178 beispielsweise eine Verbindung der Kontakte 180 umfassen. So können beispielsweise die Gegenelektrode und die Referenzelektrode sowie die Referenzelektrode und die Arbeitselektrode und optional auch die Gegenelektrode und die Arbeitselektrode miteinander verbunden werden, beispielsweise über ohmsche Widerstände, Dioden, Kapazitäten oder Kombinationen der genannten und/oder anderer Elemente. Ein derartiger ESD-Schutz 178 ist dem Fachmann grundsätzlich bekannt. Weiterhin können geeignete Kompensationsvorrichtungen für eine elektrostatische Aufladung vorgesehen sein, wie beispielsweise eine Erdung, Entladenetzwerke oder ähnliches. Wiederum alternativ oder zusätzlich können in dem Ausführungsbeispiel gemäß Figur 6 oder auch in anderen Ausführungsbeispielen auch mindestens eine Erdung und/oder elektrische Masse und/oder eine Verbindung mit derartigen Elementen vorgesehen sein. Dies ist in Figur 6 exemplarisch dargestellt. So können beispielsweise das Behältergehäuse 160 und/oder andere Komponenten des Schutzbehälters 158, beispielsweise die mindestens eine optionale und in Figur 6 angedeutete Komponente des ESD-Schutzes 178, über mindestens eine Erdungsleitung 179 geerdet sein.

Alternativ oder zusätzlich kann der Schutzbehälter 158 das Reusable 126 während der Lagerung und der Entnahme gegenüber mechanischen Einwirkungen, wie beispielsweise Stoß, Schock, Vibration oder ähnlichem Schützen oder derartige mechanische Einwirkungen zumindest abmildern. Dementsprechend kann beispielsweise eine Polsterung, eine Federung oder ähnliches in dem Schutzbehälter 158 vorgesehen sein.

Der Schutzbehälter 158 kann zur Zwischenlagerung eines bereits verwendeten Reusables 126 eingesetzt werden. Alternativ oder zusätzlich kann der Schutzbehälter 158 auch als Verpackung eines Neuproduktes verwendet werden. Dabei können der Schutzbehälter 158 und/oder das Verschlusselement ein oder mehrere, insbesondere zahlreiche, Zulassungszeichen tragen und/oder Piktogramme zur Bedienung.

Neben dem rein passiven Konzept gemäß Figur 6 sind auch andere Konzepte möglich, bei welchen der Schutzbehälter 158 eine weitergehende Funktionalität aufweisen kann. Dies ist exemplarisch in Figur 8 dargestellt, wobei nicht alle der in Figur 8 dargestellten Optionen auch realisiert werden müssen.

So kann der Schutzbehälter 158 insbesondere eingerichtet sein, um Funktionsprüfungen des Reusables 126 automatisch, permanent oder auf Anweisung durch einen Benutzer durchzuführen. Die Funktionen des Schutzbehälters 158 können dabei auch in ein übergeordnetes Failsafe-Konzept eingebunden sein. Dementsprechend kann der Schutzbehälter 158 in dem in Figur 8 gezeigten Ausführungsbeispiel exemplarisch beispielsweise eine oder mehrere Schnittstellen 182 umfassen. Diese können beispielsweise Schnittestellen für eine Kommunikation mit einem Benutzer, beispielsweise ein Bedienteil und/oder ein Display, und/oder eine Datenschnittstelle 184 und/oder ein oder mehrere Bedienelemente umfassen. Weiterhin kann eine Datenverarbeitungsvorrichtung 186 umfasst sein, beispielsweise ein Microcontroller. Weiterhin kann, alternativ oder zusätzlich, der Schutzbehälter 158 eine Energieversorgung 188 umfassen. Diese kann beispielsweise eine Batterie und/oder ein Netzteil aufweisen. Wiederum alternativ oder zusätzlich kann der Schutzbehälter 158 einen oder mehrere Datenspeicher 190 umfassen. Weiterhin kann der Schutzbehälter 158 ein oder mehrere Netzwerke 192 umfassen. Diese Netzwerke können beispielsweise interne Netzwerke und/oder externe Netzwerke umfassen, wobei in letzterem Fall beispielsweise wiederum eine oder mehrere Schnittstellen zur Verbindung mit diesen Netzwerken vorgesehen sein können, beispielsweise zur Anbindung an das Internet, an einen Arztcomputer, an einen Benutzercomputer, an ein externes Netzwerk, an einen Datenmanager oder an eine andere Komponente.

Der Schutzbehälter 158 gemäß einem oder beiden der Ausführungsbeispiele in den Figuren 6 und 8 oder gemäß weiterer Ausführungsbeispiele kann auch von weiteren Komponenten, nicht nur dem Reusable 126, im transkutanen Sensorsystem 110 oder von externen Komponenten, gesteuert werden. Beispielsweise kann eine direkte Kommunikation mit einem Datenmanager erfolgen, beispielsweise eine Funkkommunikation.

Der Schutzbehälter 158 kann einen Benutzer auch unterstützen, das Reusable 126 nach einer manuellen Desinfektion, beispielsweise einer Sprühdesinfektion und/oder einer Wischdesinfektion, zwischen unmittelbar aufeinander folgenden Einsätzen auf seine Funktionsfähigkeit zu prüfen. Dies erspart dem Benutzer im Fehlerfall den Verlust eines Disposables 124, einschließlich eines transkutanen Sensors 112. Weiterhin können auch eine gute Ablüftung und/oder eine aktive Belüftung zur Trocknung vorgesehen sein.

Der Schutzbehälter 158 kann weiterhin auch eine Funktion zu einer definierten Nachschmierung des Anschlusses 142, beispielsweise der Anschlüsse 146 und/oder 148, aufweisen. Versuche haben gezeigt, dass die nachweislich am besten geeignete Dichtungskonzeption mittels O-Ringen ohne Schmierung zu Spannungen in der Steckverbindung 144 führen kann, was zu einer schlechten Dichtwirkung führen kann. Die O-Ringe befinden sich vorzugsweise auf den Steckern 114, 134 im Disposable 124. Damit ist in der Regel gewährleistet, dass die O-Ringe, bei jedem Sensor/Disposablewechsel, eine optimale Form und optimale Elastizitätseigenschaften haben. Die O-Ringe werden beispielsweise im Disposable/Sensor- Produktionsprozess geschmiert. Mit geeigneten Reservoirs in den Dichtelementen 170 können aber Sensor 142 und Steueranschluss 146 im Steuerteil 118 nachgeschmiert und werden. Eine Schmierung der O- Ringe disposableseitig und damit ggf. eine ungünstige langfristig wirkende chemische Beeinflussung der Sensoreigenschaften durch das Schmiermittel können damit entfallen.

Als Ausführungsform kann der Schutzbehälter 158 somit beispielsweise eine hermetisch dichte Box bereitstellen, welche beispielsweise einen Abschluss gegenüber Feuchtigkeit und/oder Staub bietet. Die Box kann beispielsweise aus Kunststoffen hergestellt sein. In den Schutzbehälter 158 kann mindestens ein Reusable 126 einbringbar sein, beispielsweise durch ein Einlegen oder Einstecken durch einen Benutzer. Die Konstruktion des Reusables 126 vermeidet dabei vorzugsweise das Berühren der Kontakte 180 durch den Benutzer und minimiert somit die Gefahr von ESD während der Handhabung.

Wie in Figur 6 gezeigt, kann der sensible Steckerbereich um den Anschluss 142 herum partiell geschützt sein. Dabei kann der Anschluss 142 am Reusable 126 mittels der Dichtelemente 170, welche vorzugsweise den Dichtelementen 154 in dem transkutanen Sensorsystem 110 entsprechen, verschlossen werden.

Das optionale Trockenmittel 176 in dem Schutzbehälter 158 kann für einen definierten Zeitraum vorgesehen und dimensioniert sein. Beispielsweise kann eine ausreichende Menge vorgesehen sein, um für einen definierten Zeitraum eine definierte Luftfeuchtigkeit herzustellen, beispielsweise eine Luftfeuchtigkeit in dem Innenraum 162, welche unterhalb eines vorgegebenen Werts relativer Luftfeuchtigkeit liegt.

Die Materialien des Behältergehäuses 160 können selbst biozid und/oder fungizid ausgestaltet sein. Das Behältergehäuse 160 selbst kann wiederum gut reinigbar und/oder desinfizierbar sein, um Verschleppungen von Keimen zu vermeiden.

Eine weiterführende Ausgestaltung des Schutzbehälters 158, welche beispielsweise im Rahmen des Ausführungsbeispiels gemäß Figur 8 oder ähnlichen "intelligenten" Ausgestaltungen realisiert werden kann, kann vorsehen, dass die Kontakte 180 des Reusables 126, insbesondere die Kontakte 180 des Sensoranschlusses 148, in dem Schutzbehälter 158 mit einem "Dummy-Sensor" verbunden werden. Dabei kann ein passiver oder auch ein aktiver Sensor simuliert werden, beispielsweise in Form passiver Impedanzen und/oder auch in Form einer aktiven Elektronik. Dementsprechend kann der Schutzbehälter 158 beispielsweise eine Prüfschaltung umfassen, um das Reusable 126 boxseitig anzuschließen und optional eine Funktionsprüfung des Steuerteils vorzunehmen. Beispielsweise kann eine aktive Prüfung der Impedanzverhältnisse während der Lagerung in dem Schutzbehälter 158 durchgeführt werden. Dabei können beispielsweise Diskriminierungsschwellen von ca. ± 5 % angestrebt werden.

Ein typisches Benutzungskonzept des transkutanen Sensorsystems 110 sieht, wie oben ausgeführt, vor, den elektrischen Energiespeicher 130, beispielsweise eine Batterie, und einen Datenspeicher 132, beispielsweise ein EEPROM oder Flash-EPROM, im Disposable 124 einzusetzen und über den Steueranschluss 146 und den Steuerstecker 134 mit dem Reusable 126 zu verbinden. Bei Kontakt des Reusables 126 mit dem Disposable 124 oder Bodymount 128 erfolgt die Spannungsversorgung des Reusables 126, worauf dieses beispielsweise mit einem Reset den Funktionsablauf des transkutanen Sensorsystems 110 startet. Nach dem Start nimmt das Reusable 126 Verbindung mit dem Datenspeicher 132 auf. Durch eine spezifische Codierung des Datenspeichers 132 kann das Reusable 126 von außen veranlasst werden, in verschiedenen Modi zu arbeiten. Beispielsweise kann ein Patientenmodus, ein Fertigungsmodus, ein Programmiermodus oder ein Prüfmodus vorgesehen sein. Ist ein entsprechend programmierter Datenspeicher 132 in dem Schutzbehälter 158 integriert, würde das Reusable 126 nach einem Reset unverzüglich in den spezifischen Prüfmodus eintreten. Im einfachsten Fall kann eine Messroutine gestartet werden, bei welcher das Reusable 126, beispielsweise der Sensoranschluss 148 und dort insbesondere einer oder mehrerer der Kontakte 180, mit einem Prüfstrom beaufschlagt wird. Beispielsweise kann das Netzwerk 192 in Figur 8 hierfür eingesetzt werden. Beispielsweise kann der durch das Netzwerk 192 eingeprägte Strom gemessen und gespeichert werden. Im Fall von Leckströmen würde der gemessene Wert beispielsweise von der Vorgabe abweichen. Die Messung kann beispielsweise permanent oder in längeren zeitlichen Intervallen erfolgen. Die Speicherung kann beispielsweise in einem Datenspeicher des Reusables 126 erfolgen. Die so im Reusable 126 gespeicherten Werte können beispielsweise später beim Starten einer eigentlichen Messung mit einem transkutanen Sensor 112 vom Reusable 126 zu einer Prüfung herangezogen werden und gegebenenfalls in eine Fehlermeldung umgewandelt werden, beispielsweise derart, dass die bei der Messung erfassten Messwerte mit einer speziellen Fehlermeldung versehen werden. Diese Fehlermeldungen können bei einem Datenaustausch zwischen dem Reusable 126 und einem Datenmanager (in den Figuren nicht dargestellt) dort zur Anzeige gebracht werden und beispielsweise einen Warnhinweis oder ähnliches ausgeben, da in der Regel das Reusable 126 selbst keine eigentliche Möglichkeit aufweist, um Systemzustände visuell oder auf andere Weise, beispielsweise akustisch, anzuzeigen.

In einer komplexeren Ausführungsform, welche ebenfalls beispielsweise im Rahmen der Figur 8 realisierbar wäre, könnte der Schutzbehälter 158 auch eine eigene Intelligenz in Form einer Datenverarbeitungsvorrichtung 186 aufweisen. Diese kann beispielsweise einen Microcontroller (µC), einen oder mehrere Datenspeicher, Peripheriegeräte, Firmware oder Kombinationen der genannten und/oder anderer Elemente aufweisen. Damit können weiterführende messtechnische Prüfungen durchgeführt werden. Informationen zwischen der Intelligenz des Schutzbehälters 158 und dem Reusable 126 können über den Steuerverbinder 168 und den Steueranschluss 146 unidirektional oder auch bidirektional ausgetauscht werden. Diese Schnittstelle zwischen Steuerverbinder 168 und Steueranschluss 146 kann beispielsweise einen SPI, einen I2C oder einen 1-Draht-Bus umfassen. Auf diese Weise können nicht nur Fehlerstatistiken bezüglich der Reusable-Messung sondern auch weitere Diagnoseinformationen beispielsweise später im Datenmanager sichtbar gemacht werden, wenn der Inhalt des Datenspeichers des Reusables 146 vollständig oder teilweise an diesen Datenmanager übermittelt wird. Das System kann auch derart erweitert werden, dass Informationen vom Datenmanager an das in dem Schutzbehälter 158 befindliche Reusable 126 übermittelt werden und vom Reusable zum Schutzbehälter 158. So können beispielsweise über den Anschluss 142 Informationen zum Schutzbehälter 158 übermittelt werden, so dass der Schutzbehälter 158 gegebenenfalls von einem Benutzer konditioniert werden kann und beispielsweise mit einem Update, beispielsweise einem Software-Update, versehen werden kann. Diese Möglichkeit kann alternativ oder zusätzlich zur Verwendung der oben beschriebenen optionalen Datenschnittstelle 184 des Schutzbehälters 158 erfolgen.

Es ist aber auch möglich, dem Schutzbehälter 158 selbst mit einer eigenen Bedienoberfläche auszustatten. Mit dieser können Einstellungen unterschiedlicher Art über Bedienelemente vorgenommen werden. So kann beispielsweise ein Display vorgesehen sein, welches eine einfache LED-Anzeige, ein segmentiertes und/oder alphanumerisches Display, ein Matrix-Display oder andere Arten von Anzeigenelementen oder Kombinationen der genannten oder andere Anzeigeelemente umfassen kann.

Wie oben dargestellt, kann das Reusable 126, wenn dieses in den Schutzbehälter 158 eingelegt ist, als bidirektionaler Kommunikationskanal vom und/oder zum Datenmanager fungieren. Display und Eingabefunktionen des Datenmanagers können damit als Bedienkonsole auch für den Schutzbehälter 158 verwendet werden. Der Schutzbehälter 158 kann jedoch, alternativ oder zusätzlich, auch direkt mit dem Datenmanager kommunizieren. Dies kann beispielsweise über die Datenschnittstelle 184 erfolgen. Beispielsweise kann diese Kommunikation eine Funkkommunikation umfassen. Auch auf diese Weise können die Bedienoberfläche und/oder das Display des Datenmanagers genutzt werden, um die Funktionalität und/oder Konfiguration des Schutzbehälters 158 zu beeinflussen.

Da insbesondere in den letzteren Fällen der Schutzbehälter 158 eine höhere Menge an Energie benötigt, ist der Schutzbehälter 158 dann vorzugsweise mit einer entsprechend großen Energieversorgung 188 ausgestattet, beispielsweise mindestens einer Primärbatterie und/oder einer Sekundärbatterie, oder sogar, alternativ oder zusätzlich, mit einem Netzanschluss versehen. Dieser kann zur direkten Stromversorgung dienen, kann jedoch auch als Ladegerät für den mindestens elektrischen Energiespeicher fungieren. Damit kann der Schutzbehälter 158 sowohl mobil als auch stationär eingesetzt werden.

Weitere mögliche Ausgestaltungen betreffen die oben beschriebene Funktionsprüfung. So kann ein Prüfablauf aus einer einzelnen Leckstrom-Messung bestehen. Alternativ oder zusätzlich kann der Prüfablauf auch eine Erfassung eines Werte-Continuums über eine definierte Zeit hinweg umfassen, um aus diesen zeitbezogenen Informationen eine weiterreichende Diagnose und/oder gegebenenfalls differenziertere Informationen oder Meldungen abzuleiten. Auf diese Weise können beispielsweise Widerstandsänderungen über die Zeit hinweg (beispielsweise durch Alterungs- oder Verschmutzungseffekte), Artefakte oder ähnliche Effekte detektiert werden.

Ferner kann der Schutzbehälter 158 über eine Echtzeit-Information verfügen. Diese kann beispielsweise ähnliche zum Reusable 126 vom Datenmanager synchronisiert werden. Alternativ oder zusätzlich kann jedoch auch eine eigenständige Echtzeit-Uhr im Schutzbehälter 158 vorgesehen sein (Real Time Clock, RTC). Allgemein können auf diese Weise den Messwerten und gegebenenfalls Fehlermeldungen Echtzeiten zugeordnet werden, beispielsweise durch ein Anfügen in einem Datenspeicher.

In einem abgestimmten Failsafe-Konzept können die Schutzbehälter-seitigen Funktionen auch auf der Ebene des Reusables 156 gesteuert werden, so dass dann am Verbinder 164 nur ein passives Netzwerk erforderlich ist. In diesem Fall sind Prüfungen in der Regel nur möglich, wenn das Reusable 126 vollumfänglich funktionsfähig ist.

Hat die Box jedoch eine eigene Intelligenz und einen eigenständigen Kommunikationskanal, so können Leckstrombestimmungen auch unabhängig von der Reusable-Elektronik bestimmt und gemeldet werden. Ein solches redundantes System erhöht die Sicherheit weiter. Über Leckstrommessungen hinaus können auch beispielsweise Spannungen an der Referenzelektrode und/oder der Gegenelektrode simuliert und/oder geprüft werden.

Der optionale Kommunikationskanal zum Schutzbehälter 158, beispielsweise die Datenschnittstelle 184, welche drahtlos oder auch drahtgebunden ausgestaltet sein kann, kann unidirektional oder vorzugsweise bidirektional ausgestaltet sein. Diese Datenschnittstelle 184 kann drahtgebunden oder auch drahtlos realisiert werden, letzteres beispielsweise optisch oder auf Funk basierend. Auch ein Internetanschluss ist möglich.

Wird das passive Netzwerk des Schutzbehälters 158 durch eine oder mehrere aktive Stromquellen und/oder Spannungsquellen ersetzt und/oder ergänzt, so kann auch beispielsweise eine Nachjustage des Reusables 126 durchgeführt werden. Diese Nachjustage kann alternativ oder zusätzlich zu einer Kalibration erfolgen, wobei der Begriff der Kalibration üblicherweise im Zusammenhang mit einer Referenzierung auf eine andere Art der Messung der Analytkonzentration bezogen wird, beispielsweise auf eine Referenzierung auf Vollblut mittels eines oder mehrerer Testelemente, beispielsweise Teststreifen.

Auch eine Kalibration oder Rekalibration ist alternativ oder zusätzlich mittels des Schutzbehälters 158 prinzipiell möglich. So kann beispielsweise das transkutane Sensorsystem 110 relativ zu Vollblut kalibriert werden, das mit einem so genannten Spot-Messgerät separat ermittelt wird. Dieses kann beispielsweise ein Teststreifen-Messgerät oder eine andere Art von Messgerät zum Nachweis des mindestens einen Analyten umfassen, welches beispielsweise integriert in den Datenmanager ausgebildet sein kann oder auch separat ausgebildet sein kann. Diesbezüglich kann beispielsweise wiederum auf US 2008/0242962 A1 verwiesen werden. Alternativ oder zusätzlich kann auch in dem Schutzbehälter 158 ein derartiges Messgerät integriert sein. Beispielsweise kann in dem Schutzbehälter 158 ein Messgerät mit einem auswechselbaren Bezugssensor-Element integriert sein, das beispielsweise mit einer definierten Glucosereferenzlösung oder Analytreferenzlösung benetzt werden kann. Das Bezugssensor-Element kann dann über den Verbinder 164 und den Anschluss 142 mit dem Reusable 126 verbunden werden und der gemessene Wert im Reusable 126 dem definierten Glucosewert (oder allgemein einer Analytkonzentration) zugeordnet werden. Auch wäre ein vollständiges, in den Schutzbehälter 158 integriertes Messsystem denkbar, beispielsweise ein Vollblutmesssystem. Damit könnte beispielsweise ein auswechselbarer Sensor des integrierten Messsystems mit Vollblut bzw. einer anderen Art von Körperflüssigkeit benetzt und beispielsweise seriell an ein internes Messsystem des Schutzbehälters 158 angeschlossen werden und über dieses wiederum über den Verbinder 164 und den Anschluss 142 mit dem Reusable 126. Beispielsweise könnte ein Quotient aus beiden Messungen anschließend als Kalibrationsbasis für das transkutane Sensorsystem 110 dienen.

Da im Rahmen der obigen Beschreibung eine Vielzahl unterschiedlicher Komponenten auftreten und eine Vielzahl unterschiedlicher Begriffe für Gruppen dieser Komponenten verwendet werden, sollen diese Begriffe im Folgenden nochmals zusammengefasst werden.

Unter einem transkutanen Sensorsystem 110 wird im Rahmen der vorliegenden Erfindung allgemein ein Gruppe von Systemkomponenten, seien sie nun miteinander mechanisch und/oder elektrisch verbunden oder nicht, verstanden, welche mindestens einen transkutanen Sensor 112 und mindestens ein wieder verwendbares Steuerteil 118 sowie optional mindestens eine Steuerkomponente 120 und mindestens ein Befestigungselement 122 umfasst.

Unter dem Reusable 126, welches auch als wieder verwendbares Steuerteil 118 bezeichnet wird (beide Begriffe werden weitgehend synonym verwendet) wird eine Komponente verstanden, welche zumindest eine Elektronik zur Ansteuerung und/oder Auswertung des transkutanen Sensors 112 umfasst. Diese Komponente kann beispielsweise eine Elektronik zum Messen des Sensorstroms, zum Steuern des transkutanen Sensors 112 und/oder zum Kommunizieren mit einer Peripherie umfassen, beispielsweise mit einem Datenmanager. Der Datenmanager, welcher in den Figuren nicht dargestellt ist, kann ebenfalls Bestandteil des transkutanen Sensorsystems 110 sein. Das Reusable 126 kann beispielsweise für bis zu 50 Verwendungen eingerichtet sein.

Allgemein sei darauf hingewiesen, dass der Begriff "transkutanes" Sensorsystem auf die Verwendung eines transkutanen Sensors 112 hinweist, also eines Sensors, welcher für die Messung innerhalb eines Körpergewebes eingerichtet ist. Der Begriff "transkutan" kann somit sowohl vollständig implantierte Sensoren umfassen, welche keine drahtgebundene oder sonstige materielle Verbindung durch die Haut des Benutzers aufweisen, als auch Sensoren, bei welchen mindestens eine derartige Verbindung durch die Haut des Benutzers hindurchgeführt ist.

Unter dem Disposable 124 ist im Rahmen der vorliegenden Erfindung allgemein eine Gruppe von zusammenhängenden oder getrennt ausgebildeten Teilen zu verstehen, welche nur für einen Messzyklus verwendet werden sollten. Beispielsweise kann diese Gruppe den transkutanen Sensor 112, eine Insertionsnadel und/oder ein Insertionsbesteck, die Steuerkomponente 120 und/oder Kombinationen der genannten und/oder anderer Einweg-Elemente umfassen.

Unter dem Bodymount 128 kann allgemein die funktionelle Gruppe verstanden werden, welche die Steuerkomponente 120 sowie gegebenenfalls das Befestigungselement 122 umfasst. Die funktionelle Einheit aus transkutanem Sensor 112, Bodymount 128 und Reusable 126 kann auch als Patch bezeichnet werden.

Weiterhin wird im Rahmen der vorliegenden Erfindung die Gruppe, welche den Schutzbehälter 158 und mindestens ein Reusable 126 umfasst, als "Kit" 156 bezeichnet. Die Gruppe, welche das Kit 156 sowie mindestens einen transkutanen Sensor 112 und optional mindestens eine Steuerkomponente 120 sowie, alternativ oder zusätzlich, mindestens einen Datenmanager umfasst, wird auch als Sensorvorrichtung 157 bezeichnet. Diese kann somit das transkutane Sensorsystem 110 sowie zusätzlich den mindestens einen Schutzbehälter 158 umfassen.

In Figur 9 ist nochmals ein Verfahren zur Funktionsprüfung des Reusables 126 dargestellt, welches beispielsweise anhand der in der Figur 8 dargestellten Ausgestaltung oder im Rahmen anderer Ausgestaltungen des Schutzbehälters 158 durchgeführt werden kann. In einem Schritt 194 wird zunächst das Reusable 126 in den Schutzbehälter 158 eingebracht, beispielsweise durch ein Einlegen oder ein Stecken. Dabei kann beispielsweise die Steckverbindung 144 hergestellt werden. Anschließend wird in Schritt 196 die Funktionsprüfung gestartet. Dieser Start kann automatisch, beispielsweise durch das Einbringen selbst oder manuell, beispielsweise durch einen Benutzer, oder durch ein externes Startsignal initiiert werden. Dabei kann beispielsweise das Reusable 126 in einen Box-Modus versetzt werden.

Anschließend wird die eigentliche Funktionsprüfung durchgeführt. Diese kann beispielsweise, wie oben beschrieben, eine Leckstrommessung und/oder eine Widerstandsmessung umfassen. In Schritt 198 kann beispielsweise entschieden werden, ob die Ergebnisse dieser Funktionsprüfung einer vorgegebenen Norm entsprechen, beispielsweise ob die Ergebnisse innerhalb eines oder mehrerer Normbereiche liegen oder beispielsweise einen oder mehrere Schwellwerte überschreiten. Beispielsweise können ein oder mehrere Widerstände und/oder ein oder mehrere Leckströme mit einer oder mehreren Schwellen verglichen werden.

Ist das Ergebnis in Ordnung (Zweig 200) so kann beispielsweise abgefragt werden, ob eine Entnahme folgen soll. Ist dies nicht der Fall (Zweig 204), so kann die Funktionsprüfung in Schritt 198 wiederholt werden. Soll hingegen eine Entnahme erfolgen (Zweig 206), so kann das Reusable 126 dem Schutzbehälter 158 entnommen und einer regulären Messung 208 in einem transkutanen Sensorsystem 110 zugeführt werden.

Wird hingegen in Schritt 198 festgestellt, dass das Ergebnis nicht in Ordnung ist und somit nicht der Norm entspricht (Zweig 210), so kann in Verfahrensschritt 212 ein Fehlerstatus in dem Reusable 126 hinterlegt werden, beispielsweise in einen nicht-flüchtigen Datenspeicher wie einem EEPROM oder Flash-EPROM des Reusables 126. Anschließend kann wieder eine Abfrage 214 erfolgen, ob eine Entnahme vorgenommen wird oder nicht. Ist dies nicht der Fall (Zweig 216), so kann die Funktionsprüfung wiederholt werden. Ist dies hingegen der Fall und wird das Reusable 126 entnommen (Zweig 218), so kann die Fehlermeldung über den Fehlerstatus, der in Schritt 212 hinterlegt wurde, an den Datenmanager übermittelt werden. Dort kann dieser Fehlerstatus beispielsweise genutzt werden, um eine Messung zu verhindern, Messwerte als unsicher zu charakterisieren, einen Benutzer zu warnen oder um andere Arten von Fehlerroutinen zu initiieren.

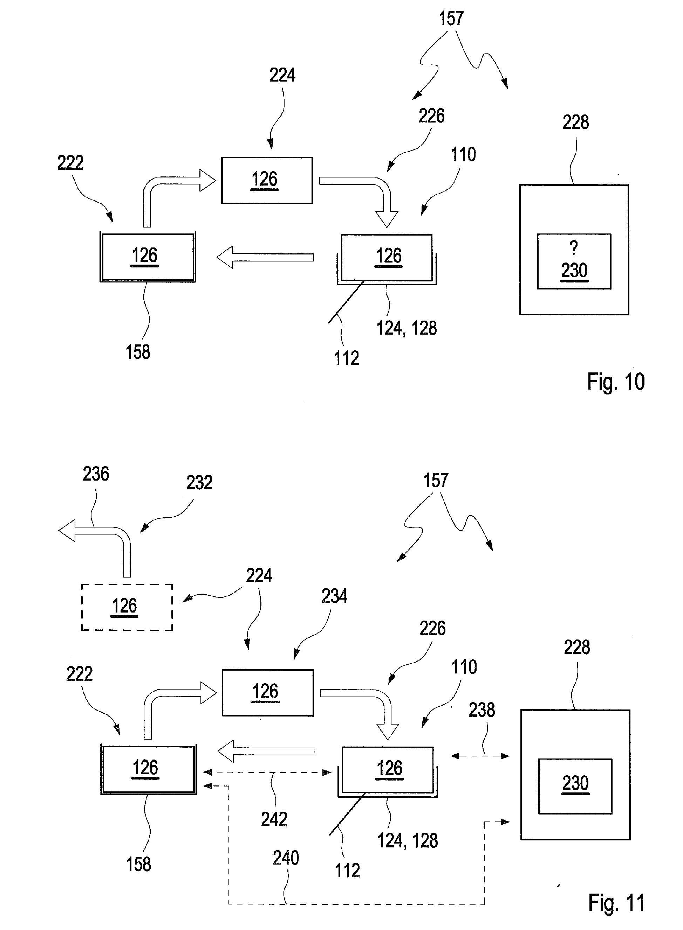

In den Figuren 10 und 11 sind zwei Ausführungsbeispiele von Verfahren zum Schutz des Reusables 126 und zum Betrieb der Sensorvorrichtung 157 in zwei Ausgestaltungen gezeigt. Das Reusable 126 ist dabei in einem Kreislauf dargestellt, in welchem dieses einmal im Schutzbehälter 158 aufgenommen ist (Position 222), einmal im freien, unverbundenen Zustand (Position 224) und einmal im transkutanen Sensorsystem 110 aufgenommen ist, beispielsweise gemeinsam mit dem Sensor 112 und dem Bodymount 128. Die Pfeile deuten an, dass der Wechsel zwischen den Positionen 222, 224 und 226 wiederholt auftreten kann.

Weiterhin ist in den Figuren 10 und 11 jeweils ein Datenmanager 228 dargestellt. Dieser Datenmanager 228 kann beispielsweise drahtlos von dem wieder verwendbaren Steuerteil 118 Daten abfragen, beispielsweise wenn dieses in das transkutane Sensorsystem 110 integriert ist. Hierfür kann beispielsweise der oben beschriebene Telemetriebaustein 140 verwendet werden. Der Datenmanager 228 kann beispielsweise ein Anzeigenelement 230 umfassen, mittels dessen beispielsweise Messinformationen und/oder auch der Status des Reusables 126, beispielsweise das Ergebnis der Funktionsprüfung, angezeigt werden können. Weiterhin kann der Datenmanager 228 beispielsweise Datenbankfunktionen, Funktionen zum Anzeigen vergangener Messwerte, Speicherfunktionen, Verwaltungsfunktionen oder andere Funktionen aufweisen, welche für einen Datenmanager eines medizinischen Systems grundsätzlich bekannt sind. Weiterhin kann der Datenmanager 228, welcher Bestandteil des transkutanen Sensorsystems 110 sein kann, auch, wie oben beschrieben, mindestens ein Messgerät zum Nachweis des mindestens einen Analyten in der Körperflüssigkeit umfassen, beispielsweise ein Spot-Messgerät, beispielsweise ein Messgerät, mittels dessen der mindestens eine Analyt qualitativ oder quantitativ mittels mindestens eines Testelements, beispielsweise eines Teststreifens, nachgewiesen wird.

In Figur 10 ist ein Ausführungsbeispiel gezeigt, bei welchem der Schutzbehälter 158 lediglich passive Schutzfunktionen aufweist, beispielsweise Schutzfunktionen gegenüber Feuchtigkeit und Schmutz und optional einen ESD-Schutz. Beispielsweise kann hierbei der Schutzbehälter 158 gemäß dem Ausführungsbeispiel in Figur 6 ausgestaltet sein. Wird das Reusable 126 dem Schutzbehälter 158 entnommen (Position 224), so ist der Status dieses Reusables 126 unbekannt. Weder in dem Schutzbehälter 158 noch im Reusable 126 selbst sind Informationen über den Status, beispielsweise die Funktionalität, beispielsweise Leckströme und/oder Widerstände, gespeichert, da diese Funktionsinformationen nicht vorhanden sind. Zwar ist durch die passiven Schutzfunktionen des Schutzbehälters 158 die Wahrscheinlichkeit reduziert, dass das Reusable 126 fehlerhaft ist. Treten dennoch Fehler auf, so machen diese sich im schlimmsten Fall erst bemerkbar, wenn das Reusable 126, wie in Position 226 dargestellt, im transkutanen Sensorsystem 110 integriert ist. Der Datenmanager 228 verfügt dann, wie durch das Fragezeichen in Figur 10 ausgedrückt wird, über keinerlei Informationen hinsichtlich des Fehlerstatus des Reusables 126.

In dem Ausführungsbeispiel gemäß Figur 11 hingegen weist der Schutzbehälter 158 eine oder mehrere aktive Funktionen auf. Beispielsweise kann dieser alle oder einige der in Figur 8 gezeigten Funktionen umfassen. Beispielsweise kann der Schutzbehälter 158 selbst eingerichtet sein, um eine Funktionsprüfung durchzuführen. Wiederum alternativ oder zusätzlich kann dieser Anzeigefunktionen umfassen, beispielsweise um eine Fehlermeldung auszugeben, wenn die Funktionsprüfung Abweichungen bestimmter Funktionen, wie beispielsweise Widerstände und/oder Leckströme, von einem Normbereich indiziert. Auf diese Weise kann beispielsweise die Meldung "Error" (Fehler) oder "Pass" (kein Fehler) ausgegeben werden. Diese Ausgabe ist jedoch grundsätzlich optional. Weiterhin sind auch andere Ausgaben möglich.

Wird das Reusable 126 dem Schutzbehälter 158 entnommen (Positionen 224 in Figur 11), so besteht zum einen die Möglichkeit, dass das Reusable 126 defekt ist (Position 232), oder dass das Reusable ordnungsgemäß funktioniert (Position 234). Diese Information über den Zustand des Reusables 126 kann beispielsweise, wie oben ausgeführt, in einem nicht-flüchtigen Datenspeicher oder einer anderen Art von Datenspeicher des Reusables 126 hinterlegt sein. Wird die Fehlerhaftigkeit des Reusables (Position 232) zuvor von dem Schutzbehälter 158 selbst angezeigt, beispielsweise über ein Anzeigenelement des Schutzbehälters 158 und/oder eines mit dem Schutzbehälter 158 in Verbindung stehenden Gerätes, so kann das Reusable 126 aus Position 232 unmittelbar entsorgt werden (Pfeil 236) und/oder an einen Hersteller oder ein Serviceunternehmen zurückübersandt werden.

Wurde hingegen angezeigt, dass das Reusable 126 fehlerfrei ist (Position 234), so kann dieses in das transkutane Sensorsystem 110 eingefügt werden. Vom Datenmanager 228 aus kann eine Statusabfrage 238 des im Reusable 126 hinterlegten Status erfolgen, so dass beispielsweise auf dem Anzeigenelement 230 der Reusable-Status angezeigt werden kann. Alternativ oder zusätzlich kann auch eine Informationsübermittlung 240 zwischen dem Datenmanager 228 und dem Schutzbehälter 158 direkt erfolgen. Diese Informationsübermittlung, welche unidirektional oder bidirektional erfolgen kann, kann beispielsweise bereits in Position 222 erfolgen, während das Reusable 126 noch in dem Schutzbehälter 158 eingesetzt ist, oder auch, alternativ oder zusätzlich, zu einem späteren Zeitpunkt, bei leerem Schutzbehälter 158. Auf diese Weise kann beispielsweise eine direkte Kommunikation zwischen Datenmanager und Schutzbehälter 158 erfolgen. Der Datenmanager 228 kann somit auch beispielsweise Funktionen des Schutzbehälters 158 anzeigen und/oder beeinflussen.

Wiederum alternativ oder zusätzlich können in diesem oder auch in anderen Ausführungsbeispielen auch direkt Informationsübermittlungen 242 zwischen dem transkutanen Sensorsystem 110 und dem Schutzbehälter 158 erfolgen. Auf diese Weise kann der Schutzbehälter 158 auch vollständig oder teilweise die Funktionen des Datenmanager 228, beispielsweise eine, mehrere oder alle der oben beschriebenen Funktionen, selbst übernehmen. Der Datenmanager 228 kann somit in den gezeigten Ausführungsbeispielen oder auch in anderen Ausführungsbeispielen der vorliegenden Erfindung optional auch vollständig oder teilweise in den Schutzbehälter 158 integriert werden.

### Bezugszeichenliste

- 110: transkutanes Sensorsystem
- 112: transkutaner Sensor
- 114: Sensorstecker
- 116: Sensorbereich
- 118: Steuerteil
- 120: Steuerkomponente
- 122: Befestigungselement
- 124: Disposable
- 126: Reusable
- 128: Bodymount
- 130: elektrischer Energiespeicher
- 132: Datenspeicher
- 134: Steuerstecker
- 136: Microcontroller
- 138: Ansteuer- und Auswerteelektronik
- 140: Telemetriebaustein
- 142: Anschluss
- 144: Steckverbindung
- 146: Steueranschluss
- 148: Sensoranschluss
- 150: Loch
- 152: Bodenplatte
- 154: Dichtelement
- 156: Kit
- 157: Sensorvorrichtung
- 158: Schutzbehälter
- 160: Behältergehäuse
- 162: Innenraum
- 164: Verbinder
- 166: Sensorverbinder
- 168: Steuerverbinder
- 170: Dichtelement
- 172: Verschlusselement
- 174: Dichtelement
- 176: Trockenmittel
- 178: ESD-Schutz
- 179: Erdungsleitung
- 180: Kontakte
- 182: Schnittstelle
- 184: Datenschnittstelle
- 186: Datenverarbeitungsvorrichtung
- 188: Energieversorgung
- 190: Datenspeicher
- 192: Netzwerk
- 194: Einbringen Reusable in Schutzbehälter
- 196: Start Funktionsprüfung
- 198: Ergebnis in Ordnung?
- 200: Ergebnis in Ordnung
- 202: Entnahme?
- 204: keine Entnahme
- 206: Entnahme
- 208: reguläre Messung
- 210: Ergebnis in Ordnung
- 212: Hinterlegung Fehlerstatus in Reusable
- 214: Entnahme?
- 216: keine Entnahme
- 218: Entnahme
- 220: Übermittlung Fehlerstatus an Datenmanager
- 222: Reusable in Schutzbehälter
- 224: Reusable frei
- 226: Reusable im transkutanen Sensorsystem
- 228: Datenmanager
- 230: Anzeigeelement
- 232: Reusable defekt
- 234: Reusable ok
- 236: Entsorgung
- 238: Statusabfrage
- 240: Informationsübermittlung
- 242: Informationsübermittlung

## Patentansprüche

1. Schutzbehälter (158) zur Aufnahme eines wieder verwendbaren Steuerteils (118) eines transkutanen Sensorsystems (110) zum Nachweis mindestens eines Analyten in einer Körperflüssigkeit, wobei das Steuerteil (118) mindestens einen Anschluss (142) umfasst, wobei der Anschluss (142) mindestens einen Sensoranschluss (148) zur Verbindung mit mindestens einem transkutanen Sensor (112) aufweist, wobei der Schutzbehälter (158) mindestens ein Behältergehäuse (160) aufweist, wobei das Steuerteil (118) in dem Behältergehäuse (160) aufnehmbar ist, wobei das Behältergehäuse (160) eingerichtet ist, um das Steuerteil (118) gegenüber Umwelteinflüssen abzuschirmen, wobei das Behältergehäuse (160) weiterhin mindestens einen Verbinder (164) aufweist, wobei der Verbinder (164) mit dem Anschluss (142) verbindbar ist und diesen mediendicht verschließt.

2. Schutzbehälter (158) nach dem vorhergehenden Anspruch, wobei der Anschluss (142) weiterhin mindestens einen Steueranschluss (146) zur Verbindung des Steuerteils (118) mit mindestens einer Steuerkomponente (120) aufweist, wobei der Verbinder (164) mindestens einen Sensorverbinder (166) zum Verbinden mit dem Sensoranschluss (148) und mindestens einen Steuerverbinder (168) zum Verbinden mit dem Steueranschluss (146) aufweist.

3. Schutzbehälter (158) nach einem der vorhergehenden Ansprüche, wobei das Behältergehäuse (160) elektrisch abschirmende Eigenschaften aufweist.

4. Schutzbehälter (158) nach einem der vorhergehenden Ansprüche, wobei der Verbinder (164) eingerichtet ist, um eine elektrische Verbindung mit dem Anschluss (142) einzugehen.

5. Schutzbehälter (158) nach einem der vorhergehenden Ansprüche, wobei der Verbinder (164) eingerichtet ist, um einen ESD-Schutz (178) für das Steuerteil (118) bereitzustellen.

6. Schutzbehälter (158) nach einem der vorhergehenden Ansprüche, wobei der Schutzbehälter (158) eingerichtet ist, um eine Funktionsprüfung des Steuerteils (118) vorzunehmen.

7. Schutzbehälter (158) nach dem vorhergehenden Anspruch, wobei der Schutzbehälter (158) in einer oder mehreren der folgenden Weisen ausgestaltet ist:
- der Schutzbehälter (158) ist eingerichtet, um den Anschluss (142) mit mindestens einem Prüfstrom und/oder mindestens einer Prüfspannung zu beaufschlagen;
- der Schutzbehälter (158) ist eingerichtet, um eine Leckstrommessung zwischen mindestens zwei Kontakten des Anschlusses (142), insbesondere des Sensoranschlusses (148) und/oder eines Steueranschlusses (146), vorzunehmen.

8. Schutzbehälter (158) nach einem der beiden vorhergehenden Ansprüche, wobei der Schutzbehälter (158) eingerichtet ist, um mindestens ein Ergebnis der Funktionsprüfung an mindestens ein weiteres Gerät und/oder einen Benutzer zu übermitteln.

9. Schutzbehälter (158) nach einem der drei vorhergehenden Ansprüche, wobei der Schutzbehälter (158) eingerichtet ist, um eine Warnung an einen Benutzer und/oder ein weiteres Gerät zu übermitteln, wenn bei der Funktionsprüfung eine Fehlfunktion des Steuerteils (118) ermittelt wird.

10. Schutzbehälter (158) nach einem der vorhergehenden Ansprüche, wobei der Schutzbehälter (158) mindestens eine elektrische Energieversorgung (188) aufweist, insbesondere eine oder mehrere der folgenden Energieversorgungen (188): eine interne Energieversorgung, insbesondere mindestens eine Batterie und/oder mindestens einen Akkumulator; einen Anschluss für eine externe Energieversorgung, insbesondere einen Netzstecker und/oder einen Netzanschluss.

11. Schutzbehälter (158) nach einem der vorhergehenden Ansprüche, wobei der Schutzbehälter (158) eingerichtet ist, um mindestens eine Kalibrationsinformation zu empfangen, insbesondere eine Kalibrationsinformation von einem externen Messgerät zum Nachweis des mindestens einen Analyten mittels mindestens eines Testelements, und wobei der Schutzbehälter (158) eingerichtet ist, um die Kalibrationsinformation an das Steuerteil (118) weiterzugeben.

12. Kit (156), umfassend mindestens einen Schutzbehälter (158) nach einem der vorhergehenden Ansprüche und mindestens ein wieder verwendbares Steuerteil (118) eines transkutanen Sensorsystems (110) zum Nachweis mindestens eines Analyten in einer Körperflüssigkeit, wobei das Steuerteil (118) mindestens einen Anschluss (142) umfasst, wobei der Anschluss (142) mindestens einen Sensoranschluss (148) zur Verbindung mit mindestens einem transkutanen Sensor (112) aufweist.

13. Sensorvorrichtung (157) zum Nachweis mindestens eines Analyten in einer Körperflüssigkeit, umfassend mindestens einen transkutanen Sensor (112), wobei der transkutane Sensor (112) in ein Körpergewebe implantierbar ist, wobei der transkutane Sensor (112) eingerichtet ist, um einen Analytnachweis in dem Körpergewebe durchzuführen, wobei die Sensorvorrichtung (157) weiterhin ein Kit (156) nach einem der vorhergehenden, ein Kit (156) betreffenden Ansprüche umfasst.

14. Sensorvorrichtung (157) nach dem vorhergehenden Anspruch, weiterhin umfassend mindestens eine mit dem Sensor (112) verbindbare Steuerkomponente (120).

15. Verfahren zum Schutz eines wieder verwendbaren Steuerteils (118) eines transkutanen Sensorsystems (110) zum Nachweis mindestens eines Analyten in einer Körperflüssigkeit, wobei das Steuerteil (118) mindestens einen Anschluss (142) umfasst, wobei der Anschluss (142) mindestens einen Sensoranschluss (148) zur Verbindung mit mindestens einem transkutanen Sensor (112) aufweist, wobei das Steuerteil (118) von dem transkutanen Sensor (112) getrennt und gegenüber Umwelteinflüssen abgeschirmt wird, wobei Anschluss (142) mediendicht verschlossen wird, insbesondere unter Verwendung eines Schutzbehälters (158) nach einem der vorhergehenden, einen Schutzbehälter (158) betreffenden Ansprüche.
